# EUROPEAN PATENT APPLICATION

(11) **EP 2 420 516 A1**
(43) Date of publication of application: **22.02.2012**
(21) Application number: 11185845.2
(22) Date of filing: 03.07.2008
(51) Int. Cl.: C07K 16/42, A61K 39/00, G01N 33/50, G01N 33/53, A61K 39/385, C40B 30/06, C12N 5/0784, A61K 38/00

(54) **Rapid generation of T cell-independent antibody responses to T cell-dependent antigens**

(30) Priority: 06.07.2007 US 948296 P
(62) Divisional of application: 08826206.8
(71) Applicant: Sanofi Pasteur VaxDesign Corporation, Orlando FL 32826 (US); Virginia Commonwealth University, Richmond, VA 23298-0568 (US)
(72) Inventor: El Shikh, Mohey Eldin Moustafa, Richmond, VA 23220 (US); El Sayed, Rania, Richmond, VA 23220 (US); Szakal, Andras K., Midlothlan, VA 23112-4513 (US); Tew, John G., Mechanicsville, VA 23111 (US); Drake III, Donald R., Orlando, FL 32804 (US); Wittman, Vaughan, Ovieco, FL 32765 (US); Eatrides, Jennifer, Orlando, FL 32804 (US); Warren, William L., Orlando, FL 32828 (US)
(74) Representative: Jones, Elizabeth Louise

(57) **Abstract**

The present invention relates to a two-component vaccine system with a first component comprising an antigen and a second component comprising an immune complex of the antigen or portion thereof and its use in inducing an immune response in a subject.

## Description

### CROSS REFERENCE TO RELATED CASES

This application claims the benefit of U.S. Provisional Application Serial No. 60/948,296, filed July 6, 2007, which is incorporated by reference herein in its entirety.

### BACKGROUND OF THE INVENTION

Antigens may be characterized as T cell-dependent (TD) or T cell-independent (TI), depending on whether T cell help is needed to induce an antibody response. T-dependent antigens are typically proteins or peptides that are presented by antigen-presenting cells to T cells in the context of MHC molecules, leading to T cell activation. Activated T cells deliver contact- and cytokine-mediated signals that promote antibody production, including high affinity antibodies of multiple isotypes (Mond et al. (1995) Annu. Rev. Immunol. 13, 655-692; Lesinski & Westerink (2001) J. Microbiol. Methods 47, 135-149).

TI antigens are classified into TI types 1 and 2. The TI-1 antigens, such as LPS, are potent B cell mitogens, which function by non-specifically or polyclonally activating most B cells (Lesinski & Westerink (2001) J. Microbiol. Methods 47, 135-149). The TI-2 antigens, such as polysaccharides, are often large molecules with repeated antigenic epitopes, capable of activating the complement cascade, but lack the ability to stimulate MHC-dependent T cell help (Mond et al. (1995) Annu. Rev. Immunol. 13, 655-692). In an ideal format, TI-2 antigens are typically flexible, nondegradable, and hydrophilic, so that they interact simultaneously with multiple B cell receptors (BCRs) (Dintzis et al. (1976) Proc. Natl. Acad. Sci. USA 73, 3671-3675). The molecular structure of a classical TI-2 antigen consists of a non-immunogenic backbone exhibiting recurring immunogenic epitopes ∼95-675 Å apart. This periodicity appears to be optimal for simultaneously engaging and cross-linking multiple BCRs and rapidly (within ∼48 h) stimulating IgM responses (Dintzis et al. (1983) J. Immunol. 131, 2196-2203; Dintzis et al. (1976) Proc. Natl. Acad. Sci. USA 73, 3671-3675).

Germinal centers (GC) are microscopically distinguishable structures in secondary lymphoid tissue where antigen (Ag)-stimulated B cells are induced to rapidly proliferate, isotype switch, somatically hypermutate, and generate high-affinity antibody (Ab)-forming cells and memory B cells. Follicular dendritic cells (FDCs) reside in the light zones of germinal centers (GC) and retain Ags in the form of immune complexes (ICs). FDCs are prominent in GCs because their numerous long slender dendrites intertwine and create extensive FDC networks or reticula. These FDC networks are fixed in the follicles while T cells and B cells are free to circulate. Nevertheless, FDCs release chemokines that attract recirculating lymphocytes that help organize the follicle and participate in the GC reaction by presenting iccosomal antigen that stimulates B cells and provides antigen for GC B cells to process and present to GC CD4⁺ T cells for help. FDCs, residing in the light zones of GCs, retain antigens in the form of ICs on numerous long slender intertwining dendrites. This creates extensive antigen retaining reticula (ARR), intimately in contact with numerous mobile B cells (Szakal et al. (1989) Annu. Rev. Immunol. 7, 91-109; Szakal et al. (1983) J. Immunol. 131, 1714-1727; Qin et al. (2000) J. Immunol. 164, 6268-6275). In GCs, which are typically T-dependent hot spots involved in refining humoral immunity, FDC functions include promotion of B cell survival, Ig class switching, production of B memory cells, promoting somatic hypermutation, selection of somatically mutated B cells with high affinity receptors, affinity maturation, induction of secondary Ab responses and regulation of high affinity serum IgG and IgE (Lindhout et al. (1993) Clin. Exp. Immunol. 91, 330-336; Lindhout & de Groot (1995) Histochem. J. 27, 167-183; Liu et al. (1991) Eur. J. Immunol. 21, 1905-1910; Schwarz et al. (1999) J. Immunol. 163, 6442-6447; Tew et al. (1990) Immunol. Rev. 117, 185-211; Qin et al. (1998) J. Immunol. 161, 4549-4554; Berek & Ziegner (1993) Immunol. Today 14, 400-404; MacLennan & Gray (1986) Immunol. Rev. 91, 61-85; Kraal et al. (1992) Nature 298, 377-379; Liu et al. (1996) Immunity 4, 241-250; Tsiagbe et al. (1992) Immunol. Rev. 126, 113-141; Tew et al. (1997) Immunol. Rev. 156, 39-52; Helm et al. (1995) Eur. J. Immunol. 25, 2362-2369; Kosco et al. (1992) J. Immunol. 148, 2331-2339; Wu et al. (2008) J. Immunol. 180, 281-290).

TD antigens trapped as ICs on the surface of FDCs are displayed in a periodic manner, with a characteristic ∼200-500 Å spacing (Sukumar et al. (2008) Cell Tissue Res. 332, 89-99; Szakal et al. (1985) J. Immunol. 134, 1349-1359). This IC periodicity on FDCs has been reported *in vivo* (Szakal et al. (1985) J. Immunol. 134, 1349-1359) and *in vitro* (Sukumar et al. (2008) Cell Tissue Res. 332, 89-99). TD antigens trapped periodically as ICs on the surfaces of flexible FDC dendrites with ∼200-500 Å spacing corresponds with T-I-2 antigens with recurring immunogenic epitopes ∼95-675 Å apart on a flexible backbone (Dintzis et al. (1983) J. Immunol. 131, 2196-2203; Dintzis et al. (1976) Proc. Natl. Acad. Sci. USA 73, 3671-3675). These epitope clusters on FDC dendrites may simultaneously cross-link multiple BCRs; thus, FDCs may convert TD antigens into TI antigens, capable of inducing B cell activation and rapid IgM production in the absence of T cells or T cell factors.

Heinemann & Peters (2005) described follicular dendritic-like cells derived from human monocytes (BMC Immunol. 6, 23; see also WO 2005 / 118779 and EP 04012622.9). These FDC-like cells were derived from their presumed precursors, monocytes, in vitro. Heinemann & Peters reported a protocol for generating FDC-like cells. Using purified human monocytes as a starter population, low concentrations of IL-4 (25 U/mL) and GM-CSF (3 U/mL), in combination with dexamethasone (Dex) (0.5 µM) in serum-free medium, triggered the differentiation of monocytes into FDC-like cells. After transient de novo membrane expression of alkaline phosphatase (AP), such cells highly up-regulated surface expression of complement receptor I (CD35). Co-expression of CD68 confirmed the monocytic origin of both the AP+ and CD35+ cells. The common leukocyte antigen CD45 was strongly down-regulated. Successive stimulation with TNF-α up-regulated adhesion molecules ICAM-1 (CD54) and VCAM (CD106). Both, AP+ and AP- FDC-like cells heterotypically clustered with and emperipolesed B cells and exhibited the FDC-characteristic ability to entrap functionally preserved antigen for prolonged times.

There is long history of immunizing with immune complexes (for review, see, e.g., Brady (2005) Infection & Immunity 73, 671-678). Further, when immunizing with ICs, the response is often rapid. "The response of rhesus monkeys to Venezuela equine encephalitis vaccine was enhanced by ICs. Remarkably, sustained protection was observed in mice just 24 h after ICs that compared with responses 8 days after antigen alone." (J. Infect. Dis. 135, 600-610, 1977). Why then are ICs not the standard for immunization? In short, ICs are not the standard for immunization because profound suppression is also often observed. The product Rhogam is a good example of such suppression (see, e.g., Clynes (2005) J. Clin. Invest. 115, 25-7).

### SUMMARY OF THE INVENTION

Follicular dendritic cells (FDCs) periodically arrange membrane-bound immune complexes (ICs) of T-dependent antigens ∼200-500Å apart, leading to the suggestion that antigen in FDC-ICs can cross-link multiple B cell receptors (BCRs) and induce T cell-independent B cell activation. As an example, ovalbumin ICs on FDCs were shown to induce purified B cells *in vitro* and in anti-Thy-1 pretreated nude mice to produce ovalbumin-specific IgM within ∼48 h. Moreover, these nude mice had GL7⁺ germinal centers (GCs) with IC-retaining FDC-reticula and Blimp-1⁺ plasmablasts. Rat-anti-mouse IgD (clone 11-26), which did not activate B cells *per* se, was converted to a potent polyclonal B cell activator when loaded as ICs on FDCs. FDC-anti-IgD induced high phosphotyrosine levels in caps and patches on virtually all purified B cells and strong dose-dependent polyclonal IgM responses within ∼48 h.

The present invention comprises the use of FDCs or FDC-like cells to generate FDC-dependent, but T cell-independent, responses to T cell-dependent antigens, with antigen-specific and polyclonal antibody production in ∼48 h. In embodiments of the present invention, ICs were used to load FDCs or FDC-like cells and B cells were stimulated *in vitro* and *in vivo* in the absence of T cells or T cell factors.

An embodiment of the present invention comprises an *in vitro* germinal center (GC) lymphoid tissue equivalent (LTE) where B cells can be induced to produce specific antibodies, class switch, mutate and produce high-affinity antibodies. ICs were used to load FDCs and B cells were stimulated *in vivo* and *in vitro* in the absence of T cells or T cell factors. Our data indicated that IC-challenged nude mice produced antigen-specific IgM within ∼48 h after IC challenge and the response was maintained for many weeks. In marked contrast, antigen in adjuvant induced no antigen-specific IgM at any time. The draining lymph nodes of the IC-challenged mice exhibited well-developed PNA⁺ and GL7⁺ GCs associated with Ag-retaining reticula (ARR) and Blimp-1⁺ plasmablasts. Moreover, purified FDCs loaded with ICs induced purified human and murine B cells to produce antigen-specific IgM *in vitro* in ∼48 h. Additionally, FDCs loaded with ICs containing anti-delta Abs induced high levels of polyclonal IgM within ∼48 h when cultured with purified B cells. These anti-delta-IC stimulated B cells showed capping and patching of intracellular phosphotyrosine, indicative of B cell signaling. The intensity of phosphotyrosine labeling increased, as indicated by increased mean fluorescence intensity, as the entire B cell population shifted to the right in flow cytometry. An embodiment of the present invention comprises a method of using FDCs, or FDC-like cells, to convert TD Ags into TI Ags, capable of inducing B cell activation and Ig production in the absence of T cells or T cell factors.

In another embodiment of the present invention, CD4⁺ T cells were primed using monocyte-derived dendritic cells (DCs) to present antigen for 10 days *in vitro.* The GC LTE was used to generate specific IgM in the first week, followed by switching to IgG in response to antigens in the second week. The GC LTE may be used in predicting problems in immunizing humans when animal experiments fail to detect such problems. The GC LTE model of the present invention is a useful tool for rapid vaccine assessment.

In another embodiment of the present invention, dual forms of immunogen were used in the GC LTE, with free antigen being used with the DCs and ICs to load FDCs.

In another embodiment of the present invention, this dual immunization strategy was used *in vivo*; ICs were targeted to FDCs to initiate an early IgM response and expand the specific B cells while free antigen was injected into a different site to target DCs for T cell priming. This dual immunogen strategy resulted in rapid, specific IgM responses and enhanced IgG responses. Further, ICs promoted somatic hypermutation several days earlier in the immune response and this should lead to rapid production of high-affinity antibody. These *in vivo* results were consistent with the use of dual forms of immunogen in the GC LTE.

The dual immunization approach of the present invention has wide application in vaccine design and assessment. For example, people moving to areas with endemic disease could immunized to provide rapid IgM protection (∼24-48 h) and high-affinity IgG could also be obtained more quickly. Moreover, this immunization strategy may be useful for shortening the time need to prepare for booster immunizations and people with T cell insufficiencies may be immunized, to rapidly generate protective IgM.

In another embodiment of the present invention, poor vaccines that are not currently used may prove to be useful if given as ICs, to induce specific IgM, or in dual form, because the resulting Ab response is so much more potent. The ability of various poor vaccines to induce specific IgM as ICs can be assessed in the GC LTE of the present invention.

In another embodiment of the present invention, we established a germinal center (GC) lymphoid tissue equivalent (LTE) where B cells could be induced to produce specific antibodies (Abs), class switch, mutate, and produce high affinity antibodies. CD4⁺ T cells were primed using monocyte derived DCs to present antigen (Ag) for ∼10 days *in vitro.* Primed CD4⁺ T cells were mixed with naïve B cells and FDCs *in vitro* and media was harvested on days ∼7 and ∼14.

With the GC LTE, specific IgM was obtained in response to ovalbumin (OVA) in the first week, followed by switching to IgG in the second week. In addition to OVA, primary Ab responses with class switching were obtained using influenza and anthrax recombinant protective antigen (rPA) as specific antigens. Moreover, evidence of affinity maturation was obtained with OVA. In contrast, with HIV gp120 a strong IgM response was observed, but we did not see class switching in the second week, possibly as a result of gp120 binding to CD4 and interfering with T cell priming. The gp120-specific IgM response did not class switch and the IgM response persisted for 14 days in the GC LTE, suggesting that primed T cells capable of promoting class switching were lacking. In mice where gp120 does not bind to CD4 T cells, the murine B cells class switched and normal IgG responses were obtained.

These gp120 data illustrate how the GC LTE of the present invention may be useful in predicting problems in immunizing humans when animal experiments failed to detect such problems. Thus, the GC LTE model of the present invention is a useful tool for the rapid assessment of vaccines and vaccine candidates.

In another embodiment of the present invention, we demonstrated that T-dependent antigens, such as gp120, can be converted into T-independent antigens by presenting them as immune complexes (ICs) for FDCs to trap and arrange in a periodic fashion on their dendrites. This periodic arrangement allows for multiple BCRs to be engaged and IgM responses to T-dependent antigens to be induced in just ∼24-48 h, similar to a TI-2 antigen. These rapid T-independent responses were demonstrated both *in vitro* in GC LTEs lacking CD4⁺ T cells and in T cell-deficient animals. Moreover, in normal animals these ICs could induce IgG responses that were more than 10 times higher than the responses obtained using free antigen.

In another embodiment of the invention, we use dual forms of immunogen in the GC LTE, with antigen being used with the DCs and ICs to load FDCs. We then tested such a dual immunization strategy *in vivo*; ICs were targeted to FDCs to initiate an early IgM response and expand the specific B cells, while free antigen was injected at a different site to target DCs for T cell priming. In combination, rapid, specific IgM responses and enhanced IgG responses were induced. Further, the ICs promoted somatic hypermutation several days earlier in the immune response, leading to rapid production of high-affinity Abs. These *in vivo* results were consistent with the use of dual forms of immunogen in the GC LTE.

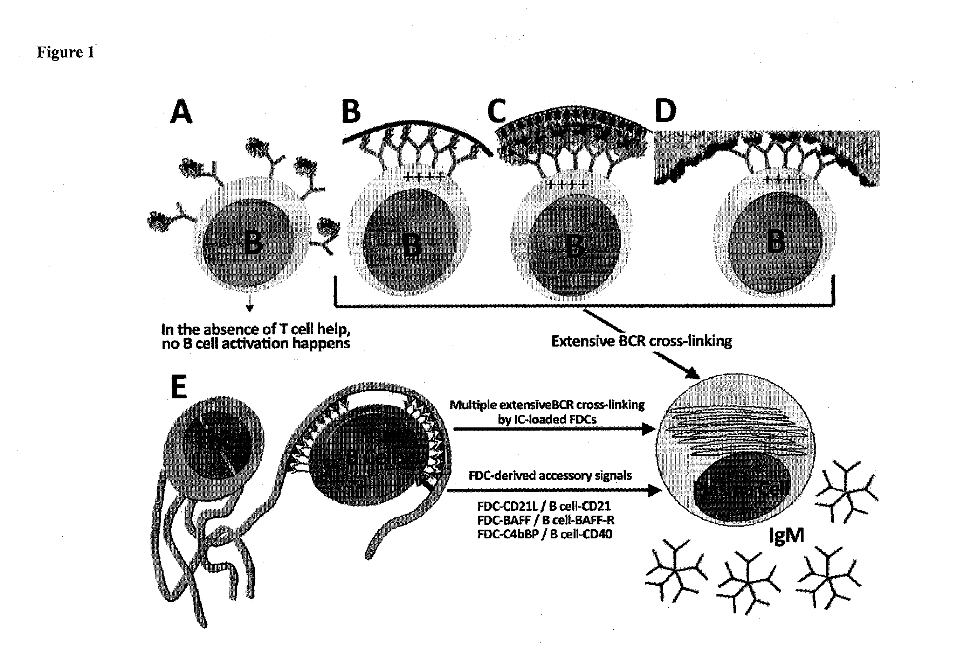

The data presented here support the novel concept that FDCs can convert TD Ags into TI Ags, capable of inducing specific IgM responses in ∼48 h or less (see Fig. 1). We reasoned that FDCs may have this ability as a consequence of observing TD-Ags in ICs on FDCs periodically spaced ∼200-500Å apart, consistent with the recurring epitopes ∼95-675Å apart on the flexible backbone of an ideal TI-2 Ag.

Thus, we suggest that the repeating epitopes of TD-Ags clustered in ICs on the surface of flexible dendrites of FDCs, or FDC-like cells, should simultaneously cross-link multiple BCRs and rapidly induce specific IgM. In contrast, free Ag, that will not decorate FDCs, or FDC-like cells, does not induce Ab responses in the absence of T cell or T cell factors, even though it would have unfettered access to BCRs.

We demonstrate here that nude mice, pre-treated with anti-Thy-1 to minimize any residual T cell activity, responded to ICs by producing specific IgM in ∼48 h while free Ag in adjuvant induced no IgM in nude mice even after many weeks. In contrast, normal mice challenged with Ag in adjuvant induced detectible IgM in 4 days, followed by IgG (data not shown) and phenotypically normal nu/+ mice injected with ICs exhibited a rapid IgM response, followed by a switch to IgG, which we attribute to T cell help that is lacking in the *nu*/*nu* mice. Moreover, the draining lymph nodes of IC-challenged nude mice exhibited well-developed PNA⁺ and GL7⁺ GCs, associated with ARR and Blimp-1⁺ plasmablasts, further supporting the concept that B cells in the follicles were stimulated by the ICs on FDCs. In contrast, GCs and plasmablasts were lacking in Ag-immunized nude controls, where the B cells remained in a resting state, consistent with the lack of T cell help.

These *in vivo* studies were consistent with *in vitro* experiments, where highly purified IC-bearing FDCs and naïve B cells from humans or mice were co-cultured in the absence of T cells or T cell factors. B cells stimulated with IC bearing FDCs in these cultures produced specific IgM in ∼48 h, while no response was observed when ICs were replaced with free Ag. Both the kinetics of the response and the IgM production are consistent with TI responses. Further, rat anti-mouse IgD mAb clone 11-26, which by itself does not activate B cells, became a potent B cell activator when made into an IC and loaded on FDCs. Activation was indicated by increased tyrosine phosphorylation in virtually all B cells, along with patching and capping. Moreover, this signaling appeared to be productive, in that FDCs bearing this IC induced polyclonal IgM in ∼48 h, consistent with a TI response. Thus, we conclude that TD Ags can induce specific IgM responses in an FDC-dependent, but T cell-independent fashion.

This concept that TD antigens can trigger B cells when appropriately arranged is supported by several literature reports. Studies on the requirements for generation of Ab responses to repetitive determinants on polymers, polysaccharides and higher order structures, such as viral capsid proteins, have indicated that high molecular weight arrays of Ag can be efficient in eliciting an Ab response independent of T-cell help, while their less ordered counterparts are less immunogenic and require T-cell help (Rosenberg (2006) AAPS J. 8, E501-E507; Vos et al. (2000) Immunol. Rev. 176, 154-170; Bachmann & Zinkernagel (1997) Annu. Rev. Immunol. 15, 235-270). Certain bacteria, viruses, mammalian cells, some polymeric proteins, such as collagen, and hapten-protein complexes have antigenic determinants in multiple repeats. The multivalent presentation of antigenic determinants extensively cross-links BCRs and leads to B cell activation, proliferation, and Ig secretion that is characteristic of TI-2 responses. For example, multimerization of monomeric proteins by aggregation facilitates presentation of their Ag determinants in a highly arrayed structure fit for cross-linking BCRs and inducing Ab responses in the absence of T cell help (Rosenberg (2006) AAPS J. 8, E501-E507).

It is important to appreciate that FDC accessory activity extends beyond delivering the primary BCR-mediated signal via Ag in the ICs. FDCs also deliver secondary or co-stimulatory signals to B cells that are important for optimal B cells activation. For example, CD21L on FDCs engages CD21 in the B cell co-receptor complex and CD21 L-CD21 interactions not only promote Ag specific responses but also polyclonal responses induced by LPS (Carter et al. (1997) J. Immunol. 158, 3062-3069; Qin et al. (1998) J. Immunol. 161, 4549-4554). In addition, FDC-BAFF and -8D6 inhibit B cell apoptosis (Li et al. (2004) Blood 104, 815-821; Ng et al. (2005) Mol. Immunol. 42, 763-772; Hase et al. (2004) Blood 103, 2257-2265; Qin et al. (1999) J. Immunol. Methods 226, 19-27; Schwarz et al. (1999) J. Immunol. 163, 6442-6447); FDCs block IC mediated ITIM signaling in B cells via FcyRIIB and minimize this inhibitory pathway (Qin et al. (2000) J. Immunol. 164, 6268-6275; Aydar et al. (2003) J. Immunol. 171, 5975-5987; Aydar et al. (2004) Eur. J. Immunol. 34, 98-107); FDCs provide IL-6 for terminal B cell differentiation (Kopf et al. (1998) J. Exp. Med. 188, 1895-1906), and FDC-C4BP engages B cell CD40 (Gaspal et al. (2006) Eur. J. Immunol. 36, 1665-1673) for a classical activation signal. Without wanting to be bound by any mechanism, we believe that the multiple accessory signals provided by FDCs make it possible to get robust IgM production in the absence of T cell help.

The short time required to get FDC-dependent TI responses may have practical application. For example, it may be important in rapidly countering infectious agents. We note a study showing protection against Venezuelan equine encephalitis just 24 h after injecting ICs; in contrast, 8 days were required for comparable protection when immunizing with free Ag (Houston et al. (1977) J. Infect. Dis. 135, 600-610). The mechanism for this rapid protection was not explained, but rapid induction of specific Ab by FDC-ICs could be the explanation for a rapid protective response after injecting ICs but not Ag.

Other applications include countering the negative effect of regulatory T cells and the non-responder state as a consequence of a limited MHC-II repertoire that may be unable to load certain peptides. Individuals who fail to respond to a vaccine, as a consequence of problems with Ag presenting cells or the effect of T regulatory cells, should mount rapid specific IgM responses when immunized with appropriate ICs. The ICs should load on FDCs and bypass limitations imposed by MHC and T cells.

Similarly, in other embodiments of the present invention, IgM responses can be induced in animals or people with congenital and/or acquired T cell insufficiencies (Grunebaum et al. (2006) Immunol. Res. 35, 117-126), including HIV-infected (Cowley (2001) Lepr. Rev. 72, 212-220), aged (Fulop et al. (2005) Drugs Aging 22, 589-603), diabetic (Spatz et al. (2003) Cell Immunol. 221, 15-26), uremic (Moser et al. (2003) Biochem. Biophys. Res. Commun. 308, 581-585), and neonatal (Garcia et al. (2000) Immunol. Res. 22, 177-190; Velilla et al. (2006) Clin. Immunol. 121, 251-259) animals or people.

The present invention comprises using follicular dendritic cells (FDCs), or FDC-like cells, to convert T cell-dependent antigens (TD Ags) into T-independent antigens (TI Ags), capable of inducing B cell activation and immunoglobulin production in the absence of T cells and T cell factors, within ∼48 hours.

Monomeric proteins generally have only a single copy of each antigenic determinant making them unable to cross-link multiple BCRs and activate B cells in the absence of MHC-restricted T cell help. The ability of FDCs to retain ICs in a periodic manner allows multimerization of these monomers and facilitates the multivalent presentation of their antigenic determinants in an array suitable for cross-linking multiple BCRs and inducing Ab responses in the absence of T cell help. Studies on the requirements for generation of Ab responses to repetitive determinants on polymers, polysaccharides and higher order structures, such as viral capsid proteins, have indicated that high molecular weight arrays of Ag are efficient in eliciting an Ab response independent of T-cell help, whereas their less ordered counterparts are less immunogenic and require T-cell help. Our results are consistent with these observations.

Moreover, these results have potential applications in preparing vaccines. For example, the negative effect of regulatory T cells and the non-responder state as a consequence of a limited MHC-II repertoire that may be unable to load certain peptides may be circumvented. Individuals who fail to respond to a vaccine, as a consequence of problems with antigen-presenting cells or the effect of T regulatory cells, should still mount rapid specific IgM responses when immunized with appropriate ICs. The ICs should load on FDCs and bypass limitations imposed by MHC and T cells. Similarly, specific IgM responses should be inducible in animals or people with congenital and/or acquired T cell insufficiencies, including HIV-infected, aged, diabetic, uremic and neonatal animals or people.

The rapidity with which FDC-dependent T cell-independent responses can be induced also has practical relevance. For example, it may be important in rapidly countering infectious or toxic agents, where a response in ∼24-48 h may be efficacious. We are impressed by studies showing protection against Venezuelan equine encephalitis ∼24 h after injecting ICs; in contrast, 8 days were required for comparable protection when immunizing with free Ag (Houston et al. (1977) J. Infect. Dis. 135, 600-610). The mechanism for this rapid protection was not explained, but rapid induction of specific Ab by FDC-ICs could explain a rapid protective response after injecting ICs, but not Ag.

The present invention is thus directed to methods for determining whether a test agent is antigenic, comprising (a) contacting an *in vitro* germinal center (GC) lymphoid tissue equivalent (LTE) with a test agent under conditions promoting production of IgM, wherein the *in vitro* GC LTE comprises B cells and follicular dendritic cells (FDCs) or FDC-like cells, wherein the follicular dendritic cells (FDCs) or FDC-like cells are loaded with immune complexes (ICs) comprising at least a portion of the test agent, and (b) assaying the *in vitro* GC LTE of (a) for IgM production, wherein when production of agent-specific IgM is found in (b), the test agent is determined to be antigenic. Preferably the B cells of the *in vitro* GC LTE are exposed to the test agent prior to contacting of the *in vitro* GC LTE with the test agent. Also preferably the test agent is a peptide, a polypeptide, a protein or a polysaccharide.

The present invention is also directed to methods for determining whether a vaccine formulation is antigenic, comprising (a) contacting an *in vitro* germinal center (GC) lymphoid tissue equivalent (LTE) with a vaccine formulation under conditions promoting production of IgM, wherein the vaccine formulation comprises at least one antigen and wherein the *in vitro* GC LTE comprises B cells and follicular dendritic cells (FDCs) or FDC-like cells, wherein the follicular dendritic cells (FDCs) or FDC-like cells are loaded with immune complexes (ICs) comprising at least a portion of the antigen comprising the vaccine formulation; and (b) assaying the *in vitro* GC LTE of (a) for IgM production, wherein when production of antigen-specific IgM is found in (b), the vaccine formulation is determined to be antigenic. Preferably, the B cells of the *in vitro* GC LTE are exposed to the antigen prior to contacting of the *in vitro* GC LTE with the vaccine. Also preferably the antigen is a peptide, a polypeptide, a protein or a polysaccharide.

The present invention is further directed to methods for determining the antigenicity of a vaccine formulation, comprising (a) contacting an *in vitro* germinal center (GC) lymphoid tissue equivalent (LTE) with a vaccine formulation under conditions promoting production of IgM, wherein the vaccine formulation comprises at least one antigen and wherein the *in vitro* GC LTE comprises B cells and follicular dendritic cells (FDCs) or FDC-like cells, wherein the follicular dendritic cells (FDCs) or FDC-like cells are loaded with immune complexes (ICs) comprising at least a portion of the antigen comprising the vaccine formulation, and (b) determining the amount of IgM produced by the *in vitro* GC LTE of (a), wherein the amount of antigen-specific IgM determined in (b) corresponds to the antigenicity of the vaccine formulation, thereby determining the antigenicity of a vaccine formulation. Preferably, the B cells of the *in vitro* GC LTE are exposed to the antigen prior to contacting of the *in vitro* GC LTE with the vaccine. Also preferably the antigen is a peptide, a polypeptide, a protein or a polysaccharide.

The present invention is additionally directed to methods for determining the antigenicity of a vaccine formulation, comprising (a) contacting an *in vitro* germinal center (GC) lymphoid tissue equivalent (LTE) with a vaccine formulation under conditions promoting production of IgM, wherein the vaccine formulation comprises at least one antigen and wherein the *in vitro* GC LTE comprises B cells and follicular dendritic cells (FDCs) or FDC-like cells, wherein the follicular dendritic cells (FDCs) or FDC-like cells are loaded with immune complexes (ICs) comprising at least a portion of the antigen comprising the vaccine formulation; (b) collecting IgM produced by the *in vitro* GC LTE of (a), and (c) determining the affinity of the antigen-specific IgM collected in (b) for the antigen, wherein the affinity of the antigen-specific IgM determined in (c) for the antigen corresponds to the antigenicity of the vaccine formulation, thereby determining the antigenicity of a vaccine formulation. Preferably the B cells of the *in vitro* GC LTE are exposed to the antigen prior to contacting of the *in vitro* GC LTE with the vaccine. Also preferably the antigen is a peptide, a polypeptide, a protein or a polysaccharide.

The present invention is also directed to methods for determining whether a two-component vaccine system is antigenic, comprising (a) contacting an *in vitro* germinal center (GC) lymphoid tissue equivalent (LTE) with a first component of a two-component vaccine system under conditions promoting production of IgM, wherein the first component of the two-component vaccine system comprises an antigen and wherein the *in vitro* GC LTE comprises B cells and follicular dendritic cells (FDCs) or FDC-like cells, wherein the follicular dendritic cells (FDCs) or FDC-like cells are loaded with immune complexes (ICs) comprising at least a portion of the antigen comprising the first component of the two-component vaccine system, (b) contacting the *in vitro* GC LTE of (a) with a second component of the two-component vaccine system under conditions promoting production of IgM, wherein the second component of the two-component vaccine system comprises the antibody and the portion of the antigen of the ICs of (a); and (c) assaying the *in vitro* GC LTE of (b) for IgM production, wherein when production of antigen-specific IgM is found in (c), the vaccine is determined to be antigenic.

In this method, preferably the B cells of the *in vitro* GC LTE are exposed to the first component of the two-component vaccine system prior to contacting of the *in vitro* GC LTE with first component of the two-component vaccine system. In a further preferred embodiment, the B cells of the *in vitro* GC LTE are exposed to the second component of the two-component vaccine system prior to contacting of the *in vitro* GC LTE with first component of the two-component vaccine system.

Also preferably, the antibody of the second component binds the portion of the antigen of the ICs of (a). Preferably the antigen is a peptide, a polypeptide, a protein or a polysaccharide.

The present invention is moreover directed to methods for generating IgM antibodies, comprising (a) contacting an *in vitro* germinal center (GC) lymphoid tissue equivalent (LTE) with an antigen, wherein the *in vitro* GC LTE comprises B cells and follicular dendritic cells (FDCs) or FDC-like cells, wherein the follicular dendritic cells (FDCs) or FDC-like cells are loaded with immune complexes (ICs) comprising at least a portion of the antigen; and (b) culturing the *in vitro* GC LTE of (a) under conditions promoting generating of IgM antibodies, thereby generating IgM antibodies.

In preferred embodiment the culturing (b) is for about 48 hours or about 72 hours. The method may also comprise collecting IgM antibodies generated in (b). The culturing (b) may continue until antibody class switching is achieved; preferably the class switching is switching from IgM production to IgG production. Also preferably the antigen is a peptide, a polypeptide, a protein or a polysaccharide.

The present invention is also directed to two-component vaccine systems comprising a first component and a second component, wherein the first component comprises an antigen and wherein the second component comprises an immune complex of the antigen of the first component. In a preferred embodiment the first component further comprises a pharmaceutically acceptable carrier or diluent and the second component further comprises a pharmaceutically acceptable carrier or diluent.

In a related embodiment the present invention is directed to methods of inducing an immune response in a subject comprising (a) administering a first component of a two-component vaccine system to a subject, wherein the first component comprises an antigen and pharmaceutically acceptable carrier or diluent; and (b) administering a second component of the two-component vaccine system to the subject, wherein the second component comprises an immune complex of the antigen of the first component, and pharmaceutically acceptable carrier or diluent.

In preferred embodiments the second component of the two-component vaccine system is administered to a different location of the subject than the first component of the two-component vaccine system. The first and second components of the two-component vaccine system may be administered concurrently or sequentially to the subject. The immune response is a rapid production of high-affinity antibodies, preferably high-affinity IgM antibodies or high-affinity IgG antibodies. In one embodiment the high-affinity antibodies are produced within about 24 hours after administration of the two-component vaccine system. In a further embodiment the immune response is a protective immune response. Preferably the antigen is a peptide, a polypeptide, a protein or a polysaccharide.

### BRIEF DESCRIPTION OF THE FIGURES

**Figure 1****.** Model of FDC-dependent T-independent B cell activation and Ig production.
   **A:** Monomeric proteins generally express only a single copy of each antigenic determinant making them unable to cross-link multiple BCRs and activate B cells in the absence of T cell help.
   **B:** TI-2 Ags contain numerous periodically arranged epitopes (green protrusions) attached to a flexible backbone (red curve). This arrangement allows extensive simultaneous cross-linking of BCRs (Y-shaped green). The multiple BCR cross-linking delivers a signal leading to B cell activation and Ig production.
   **C:** FDCs express high levels of FcyRIIB (red) and CRs (blue), which trap ICs containing TD Ags (multi-color clusters) in a periodic arrangement ∼200-500Å apart. We reasoned that this spatial arrangement would allow cross-linking of multiple BCRs specific for a single epitope, leading to B cell activation and Ig production as in panel B.
   **D:** Transmission electron micrograph showing HRP (horseradish peroxidase, a TD Ag) retained on the FDC surface in IC clusters ∼200-500Å apart. This facilitates BCR cross-linking and B cell activation as explained in panels B and C.
   **E:** FDCs accessory activity includes secondary signals or co-signals that promote B cell activation and Ig production. Specifically, FDCs are decorated with the complement-derived CD21 L which will engage B cell CD21. Binding CD21 in the CD21-CD19-CD81 complex delivers a positive co-signal for B-cell activation and differentiation, FDC-derived BAFF ligates BAFF receptors on B cells, and FDC-derived C4b-binding protein (C4BP) ligates B cell-CD40, a classical co-signal in B cell activation.
**Figure 2****.** Anti-delta IC-retaining FDCs and FDCs-like cells induce rapid (in ∼48 h) T-cell independent IgM production *in vitro.*
**Figure 3****.** OVA-specific IgM after ∼48 h in a GC LTE without CD4⁺ T cells.
**Figure 4****.** Rapid T-independent IgM response induced by ICs on FDCs. T cell-depleted *in vitro* cultures showing anti-ovalbumin (OVA) IgM production within ∼48 h of culturing naïve B lymphocytes with IC-loaded FDCs, indicating the T-independent nature of the response. However, as indicated in the final column, the presence of T cells, likely producing some cytokines, did promote the IgM response, without resulting in any IgG.
   This figure illustrates a T-independent response in the absence of any T cells. Anti-thy1 was used to remove T cells; such removal is virtually complete. An IgM response was apparent in the absence of T cells. Clearly, the IgM anti-OVA response was stronger in the presence of T cells, but it did occur in the absence of T cells.

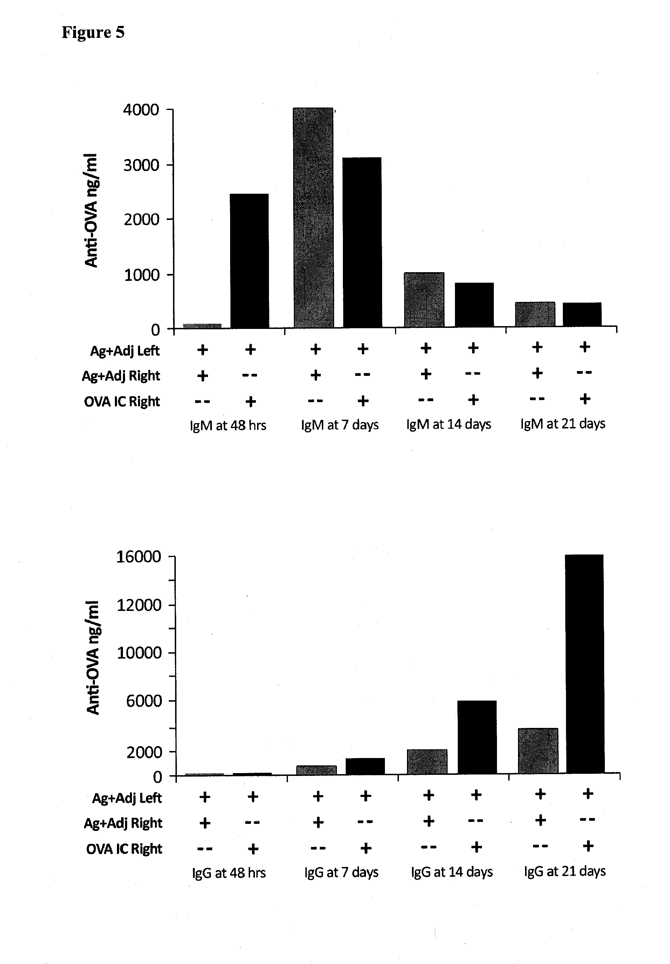

**Figure 5****.** Comparison of conventional versus dual immunogen immunization. Antigen in adjuvant would be expected to target DCs, which would lead to effective T cell priming and the ICs would be expected to target FDCs, that would select and expand specific B cell populations, such that very rapid and sustained specific Ab responses could be developed. By using such dual forms of immunogen, the promise of vaccine enhancement via ICs may finally be realized. As an example, we injected antigen in adjuvant on one side of an animal, to prime T cells, and ICs on the other side, to target FDCs, and to generate an early IgM response and get specific B cells selected and expanded. The ICs were made using OVA haptenated with NIP and anti-NIP to make the ICs such that the antibody will not interact with OVA on the T cell side of the animal and cause any feedback inhibition. The results are shown in Figure 5. Note that ICs did give a potent early IgM response at day 2, while Ag in adjuvant did not. At day 7, IgM was present for both forms of immunogen, but by day 14 the IgM response for both forms of immunogen was low, consistent with helper T cell activity and class switching. This was not seen at 14 days, or even 28 days, when only the IC was used as an immunogen. The IgG response is shown in the second panel. Note that both conventional and dual forms of immunogen gave IgG at days 7 and 14, but that the dual form of immunogen was stronger. In short, the two forms of immunogen resulted in an early IgM response and an enhanced IgG response.

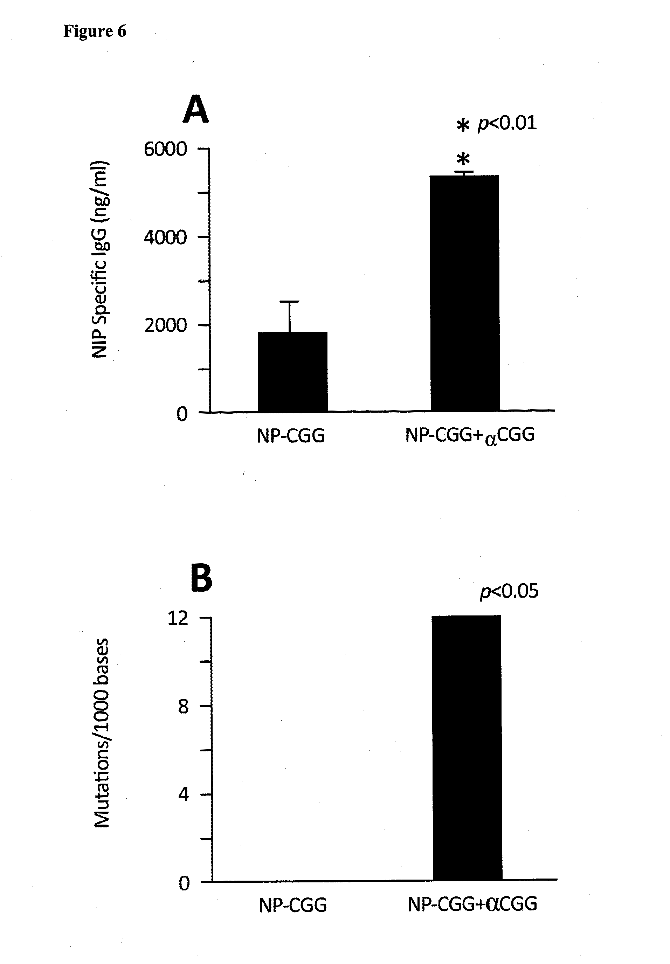

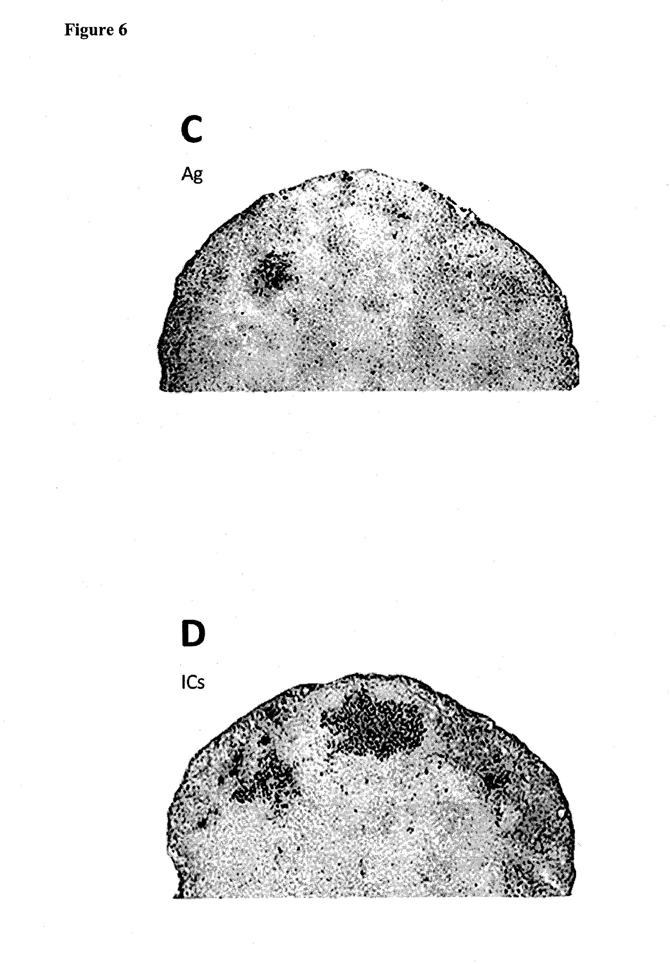

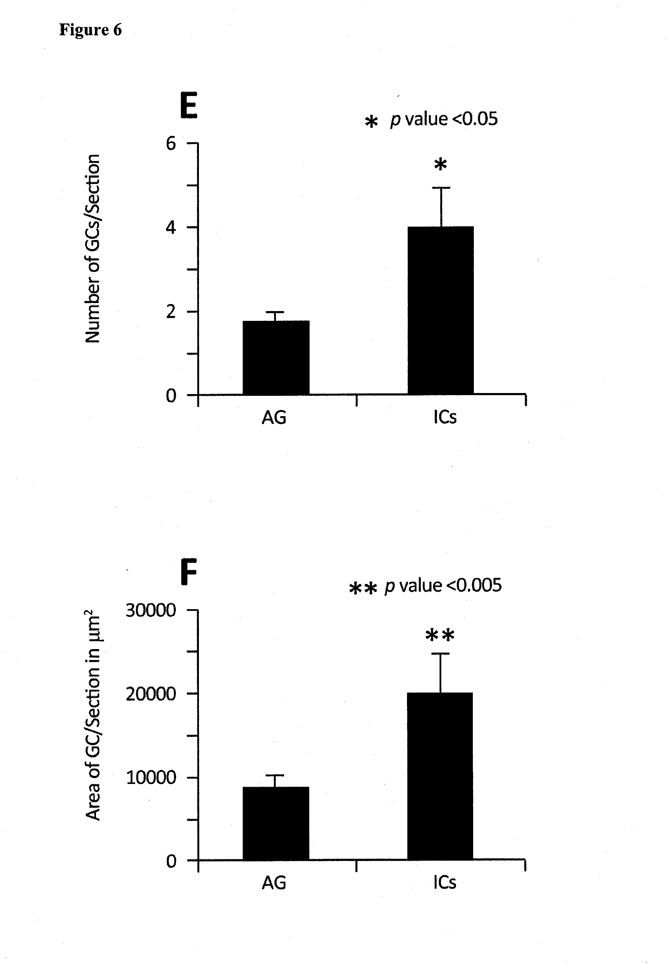

**Figure 6****.** Immune complexes promote Ab production and somatic hypermutation (SHM). Mice were irradiated with 600 rads and reconstituted with negatively selected naïve λ+ B cells and memory T (CGG) cells. These mice were divided into two groups with one receiving 5 µg of NP-CGG in preformed ICs in the hind foot pads and front legs. The control group received 5 µg of NP-CGG in each hind foot or front leg. After 7 days, λ+ B cells were isolated from lymph nodes and analyzed for VH186.2 mutations.
   **Panel a** illustrates NIP-specific IgG measured by ELISA in 3 mice per group and the error bar represents SD and the differences are statistically significant (p<0.01) and the data are representative of two experiments.
   **Panel b** illustrates the number of mutations per 1000 bases of VH186.2 clones sequenced. The λ+ B cells from the draining lymph nodes of the three mice were pooled and RNA extracted. We analyzed 10 clones in each condition and the difference in mutation frequency was statistically significant (p < 0.01).
   **Panels c & d** show representative illustrations of 10µm thin sections of draining lymph nodes from the two groups of mice labeled with anti-GL7 to identify GC B cells.
   **Panels e & f** shows cumulative data representing total number of GCs and area of GCs per mid-sagittal section of all six mice challenged with Ag or IC.

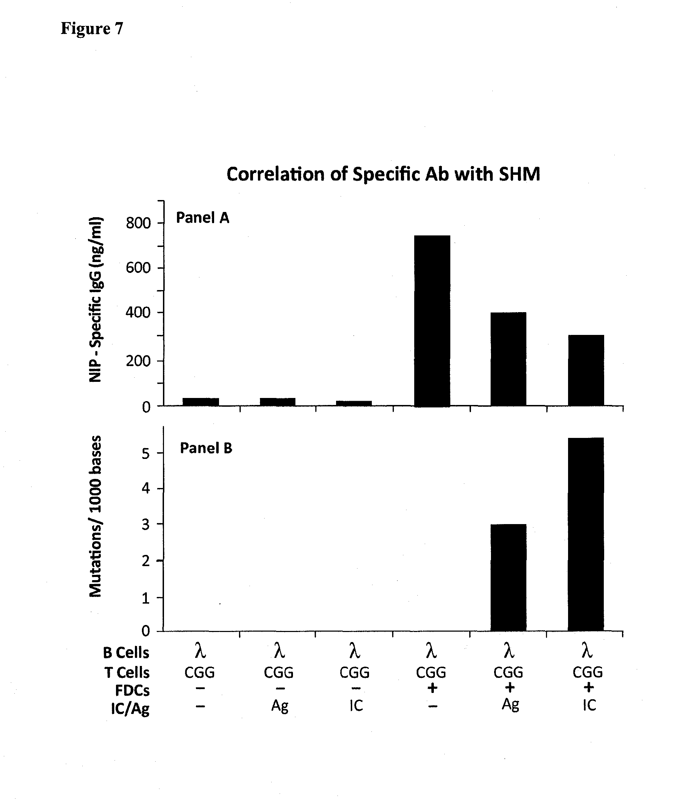

**Figure 7****.** Correlation of specific antibodies with somatic hypermutation (SHM). FDCs enhanced NIP-specific IgG production by B cells isolated 6 days after primary immunization, but SHM required FDCs plus an additional encounter with immunogen. GC reactions were initiated by culturing 1×10⁶ unmutated but 6 day primed B cells, 0.5×10⁶ T cells, and 0.5×10⁶ FDCs in the presence of 100 ng of NP(36)-CGG as free Ag or in ICs. The contents in each culture are indicated across the bottom and after 7 days of culture, supernatant fluids were collected for NIP-specific IgG assays and cell pellets were collected for RNA extraction. **Panel a** shows NIP-specific IgG production and **Panel b** illustrates mutations per 1000 bases of the VH186.2 clones recovered from the same cultures as in panel A. The rate of mutations per 1000 bases in each of the six conditions was calculated after analyzing: 10, 13, 20, 10, 14, & 14 VH186.2 clones, respectively.

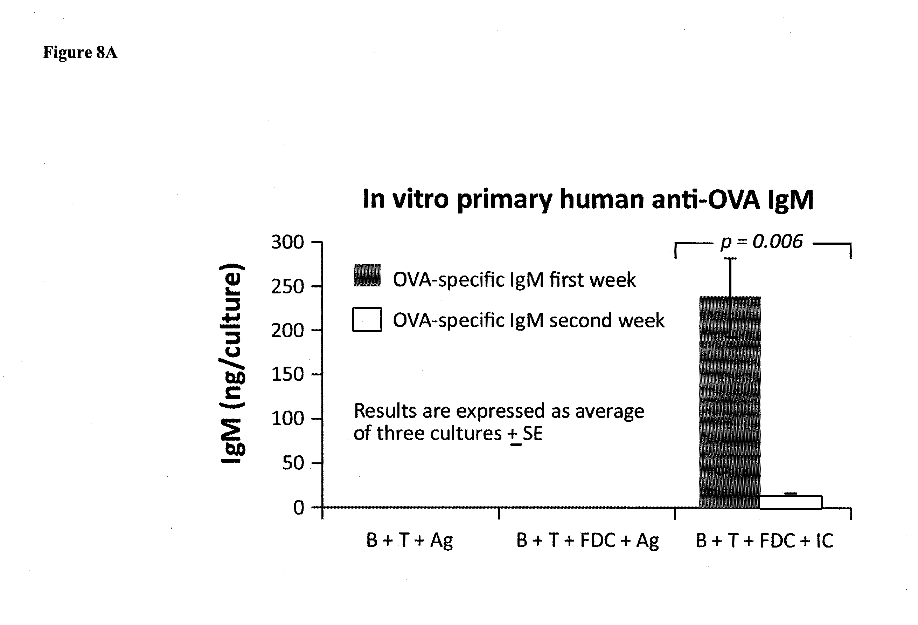

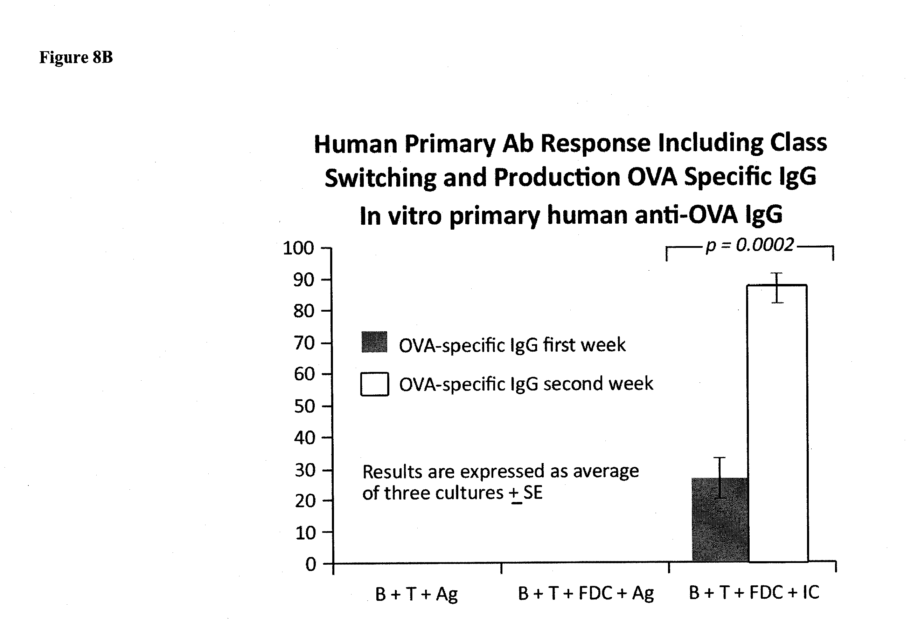

**Figure 8****.** T cells were primed with monocyte-derived DCs. Monocytes were cultured with IL-4 (1000 U/mL) and GM-CSF (800 U/mL) to generate immature DCs. After 5 days, OVA (1 µg/mL) was added to provide Ag for processing + LPS (1 µg/mL) for DC maturation. This was done in autologous serum to avoid priming for antigens in fetal calf serum. After 8 h, CD4⁺ T cells were added for OVA priming. The priming and maturation for helper T cells was allowed to go for 10 days. After priming the 4×10⁶ T cells and DCs were mixed with naïve B cells (4×10⁶ cells) and 1×10⁶ FDCs. Nine million cells total in a 24 well plate with 3 ml media in 10% autologous serum. OVA (5 µg) + murine anti-OVA (30 µg) were complexed (OVA ICs) and the ICs were placed *in vitro* to load on FDCs. Supernatant fluids were collected on days 7 and 14. Three mL of media were collected each time. **8A.** Human *in vitro* primary Ab response: production of OVA-specific IgM. **8B.** shows the IgG data. In contrast to IgM, the IgG response was low in the first week and then the response switched to all IgG in the second week. Only the combination of FDCs with ICs gave a detectible response (by ELISA).

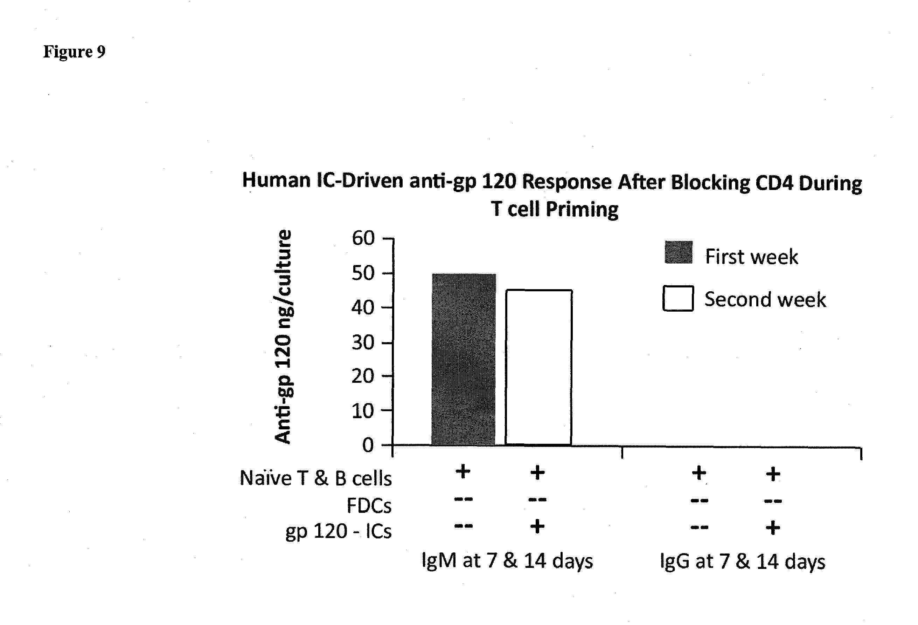

**Figure 9****.** Human IC-driven anti-gp120 response after blocking CD4 during T cell priming. A strong IgM response was seen, but class switching did not occur, as illustrated in Figure 9. We think the lack of an IgG response was likely attributable to gp120 binding CD4 and interfering with T cell priming. We attribute the strong gp120-specific IgM response to the ability of FDCs to arrange ICs on their surfaces with periodicity. This periodicity is consistent with the periodicity of independent antigens which would give the good IgM responses in the absence of primed T cells.

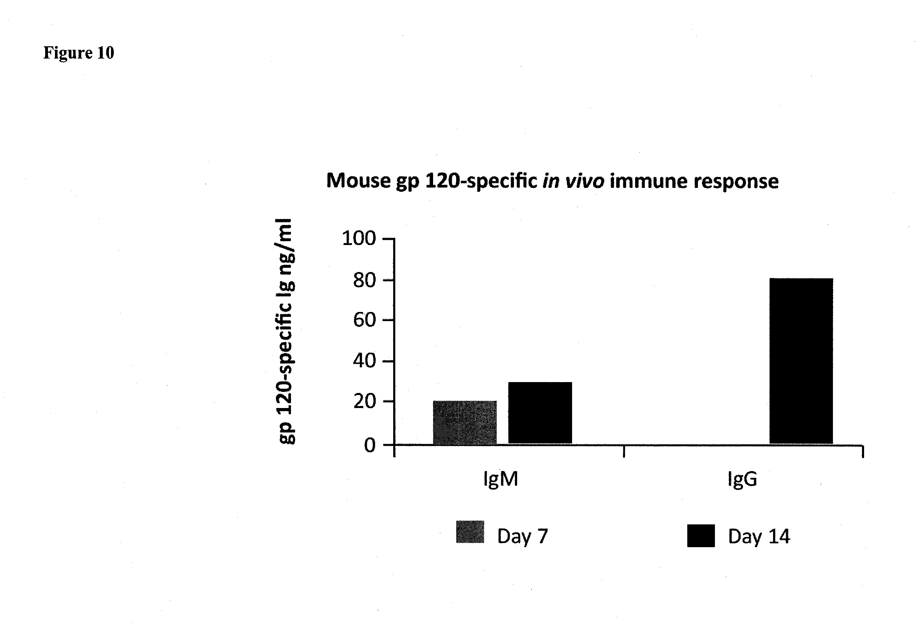

**Figure 10****.** Mouse gp120-specific *in vivo* immune response. A common way to assess potential immunogens is to start by injecting them into animals. Those immunogens that respond well in animals are candidates for further study. Consider what happens to lg class switching when gp120 was injected into mice, as illustrated in Figure 10. The murine response to gp129 was good, with IgM responses that class-switched to IgG by day 14 as expected. There was no indication that gp120 would not be a good vaccine candidate from these murine data. The GC LTE predicted problems with free, soluble gp120 that can bind human CD4 when priming human T cells and T cell help is necessary for IgG class switching. In contrast, soluble free gp120 looks like a good vaccine candidate in an animal model, where IgG class switching occurred perfectly normally. It should be appreciated that gp120 will not bind murine CD4 and would not interfere with T cell priming. Nevertheless, gp120 on the virus *in vivo* does induce a good gp120 response. Perhaps use of gp120 in a particle, mimicking the virus, might not block T cell priming as well as the free molecule that would behave more like a cytokine. Thus, designing the vaccine differently might give a different result. However, it seems unlikely that free gp120 is going to be a good immunogen in humans and only the *in vitro* GC LTE provided that information.

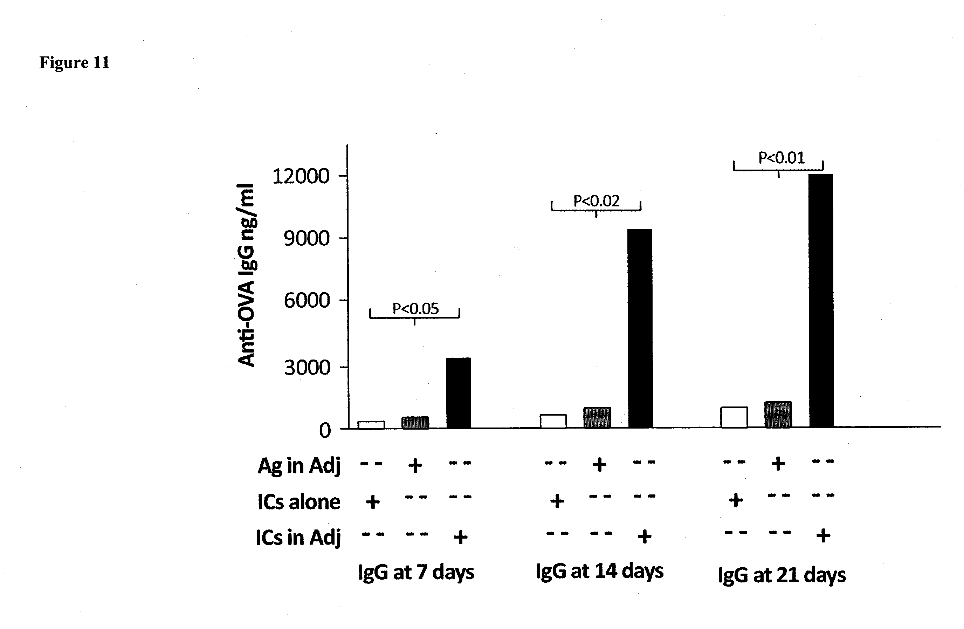

**Figure 11****.** Effect of alum-pertussis adjuvant on immunogenicy of ICs. FDCs bear TLR4 and other TLRs on their surfaces. Moreover, LPS activates FDCs and enhances their ability to stimulate antibody responses *in vitro* and promote somatic hypermutation. We examined whether adjuvant would improve the ability of ICs to promote Ab responses. The results illustrated in Figure 11 showed that ICs in adjuvant and ICs alone appeared to have comparable ability to induce OVA-specific IgG. This is an examples where the ICs were able to induce IgG without adding memory T cells or Ag to prime T cells. Nevertheless, adding adjuvant to the ICs resulted in a dramatic enhancement of the IgG responses, consistent with our data indicating that FDCs have TLR receptors and are activated by engagement of these receptors. In a preferred embodiment of the present invention, both the Ag and the ICs should be in adjuvant when immunizing with the dual immunization approach, based on the results illustrated here.

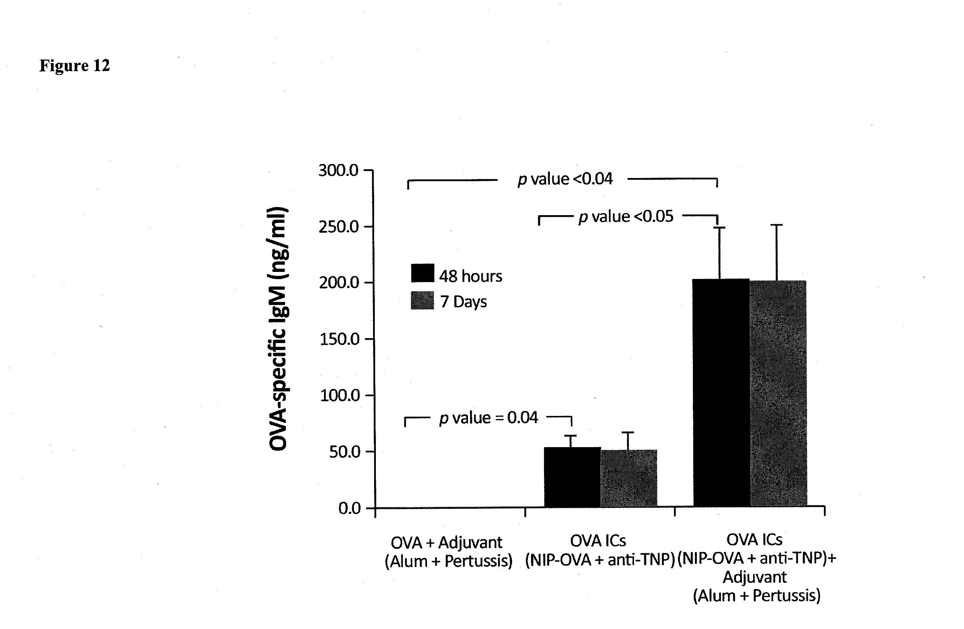

**Figure 12****.** T-dependent Ag induced IgM in nude mice and the IgM response was enhanced by use of adjuvant. IgM responses were rapid and sustained in nude mice with ICs as the immunogen (residual T cell activity was blocked with 50 µg anti-Thy-1, i.p., at the time of immunization). OVA in adjuvant failed to induce a detectible IgM response in nude mice, as was expected. In marked contrast, OVA ICs induced a significant IgM response and that response was dramatically enhanced by the use of ICs with adjuvant. These data support the concept that use of ICs may be able to provide protection in people with T cell insufficiencies where Ag fails to give a response, as illustrated here with nude mice, or a very poor response. Human immunoinsufficiencies are seen, in e.g., AIDS patients, the aged, uremics, diabetics, and alcoholics.

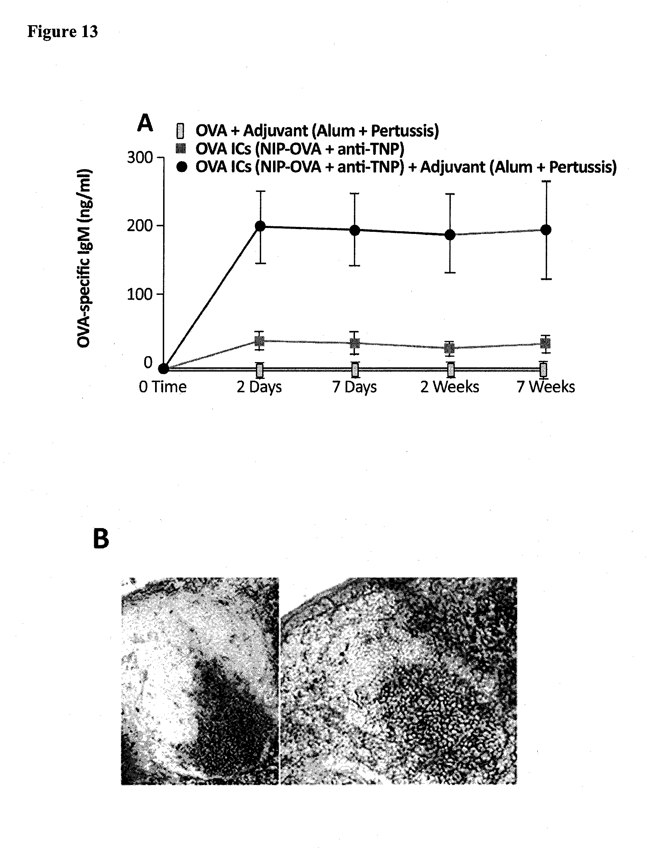

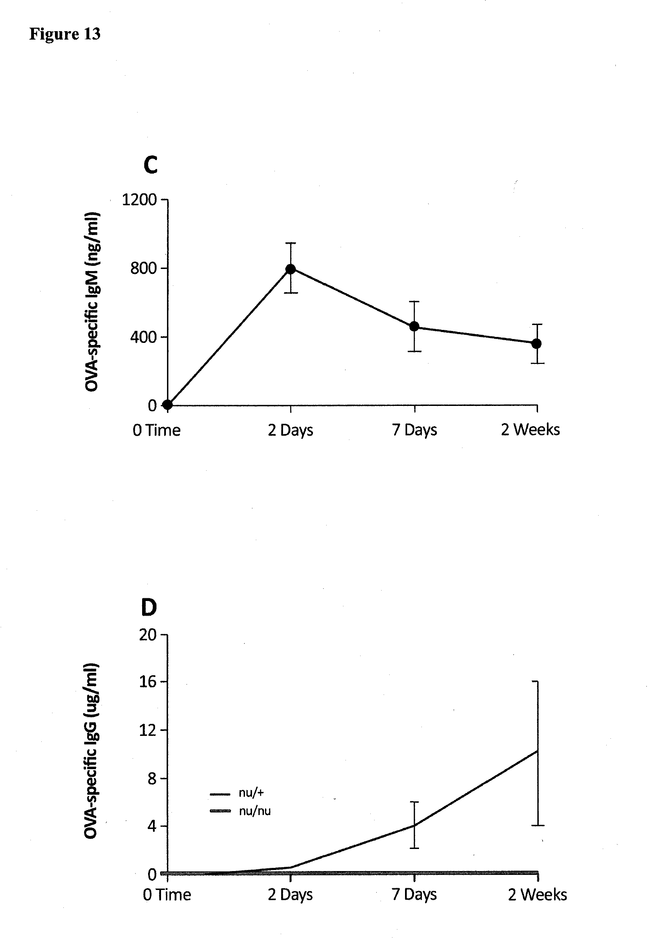

**Figure 13****.** Nude mice challenged with OVA ICs, but not with OVA, mounted OVA-specific immune responses in ∼48 h and developed GCs.
   **A**: Groups of *nu*/*nu* mice, pre-treated with 50 µg anti-Thy-1 to block residual T cell activity, were challenged with alum precipitated OVA with *Bordetella pertussis,* OVA-ICs or OVA-ICs with *Bordetella pertussis.* Serum anti-OVA IgM levels were determined 48 hours, 1 week and 2 weeks later and results were recorded after subtracting background levels using pre-immunization sera. As expected, anti-OVA was not detectible in animals immunized with OVA in adjuvant (baseline tracking). In marked contrast, OVA-specific IgM was present in the sera of all ICs injected animals with or without adjuvant in just 48 hrs and was maintained over a 7-week assessment period.
   **B**: Mid-saggittal sections from the draining popliteal lymph nodes of lC-challenged nu/nu mice were labeled for GC B cells with peroxidase-conjugated peanut agglutinin (PNA) 7 weeks after challenge with ICs. Well-developed PNA⁺ GCs were observed in these draining lymph nodes further supporting FDC-lC mediated B cell activation.
   **C**: Phenotypically normal heterozygous nu/+ mice with competent T cell compartment also responded to ICs by producing OVA-specific IgM within 48 hours, although, these IgM levels declined over time
   **D**: The phenotypically normal nu/+ also produced IgG and the increase in this isotype correlated with a decrease in IgM. Note that neither class switching nor OVA-specific IgG was detectable in nu/nu mice lacking T cell help (baseline tracking).

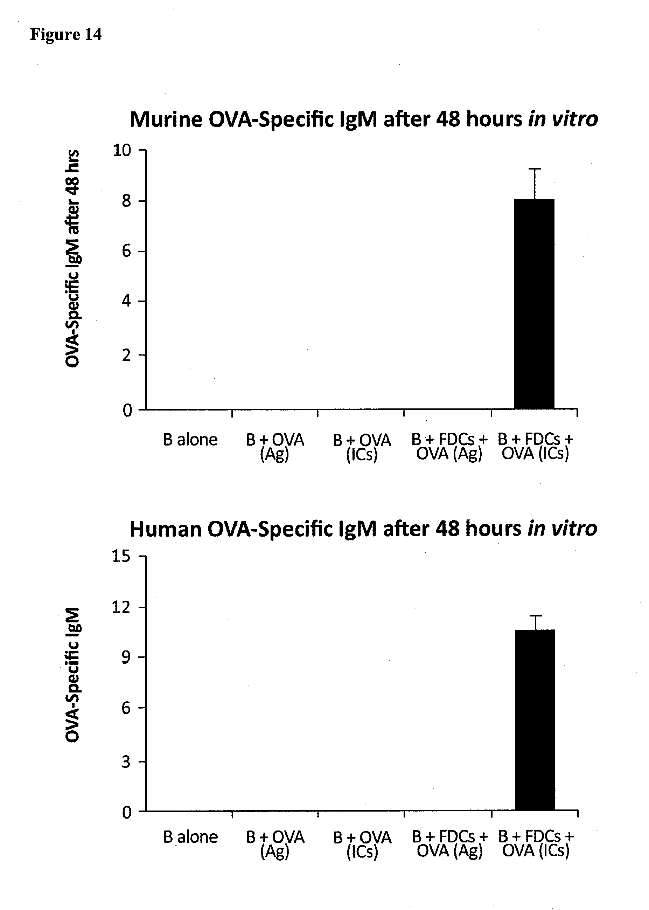

**Figure 14****.** Purified OVA-lC-bearing FDCs induced OVA-specific IgM production by purified B cells within ∼48 h in the absence of T cells. Purified murine or human B cells were incubated with purified OVA-lC-loaded FDCs at a ratio of 1FDC:2B cells and OVA-specific Abs were assessed after 48 hours. A: murine and B: human B cells. B cells stimulated with FDCs bearing OVA ICs produced OVA-specific IgM in ∼48 h. Control conditions, that failed to produce a detectable response, included FDCs with B cells stimulated with free OVA that would have had free access to BCR. The data are representative of two experiments of this type.

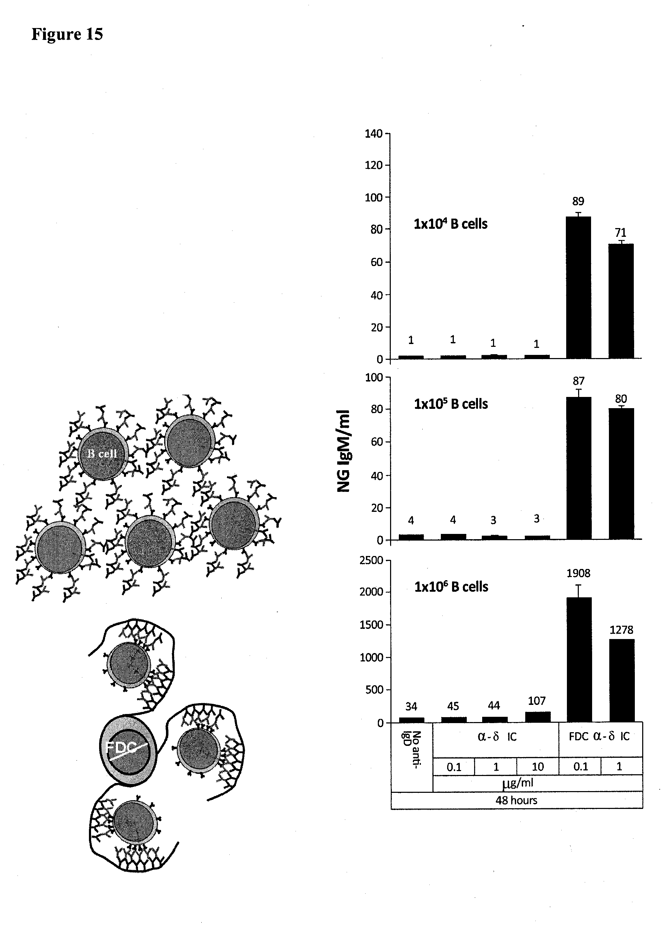

**Figure 15****.** Purified FDCs bearing anti-IgD ICs on their surfaces induced polyclonal IgM production by purified B cells within ∼48 h. Given that B cells are signaled by anti-delta ICs on FDCs, we reasoned that the simultaneous engagement of multiple B cell receptors should signal, at least some of these B cells adequately, to rapidly produce IgM (models on left). FDCs to B cells was held constant at 1FDC : 4 B cells. Rat anti-mouse IgD (mAb11-26) was held constant at 0.1 or 1 µg/mL with FDCs. The goat anti-rat to form ICs with the rat anti-lg delta was used at a ratio of 6 goat antibodies to 1 rat anti-mouse IgD mAb. The anti-lgD immune complexes in the second, third and fourth columns showed almost nothing over the level without any IgD, indicated in the first column. This was the expected result, given that there was no second signal from IL-4 or anti-CD40 for the B cell. However, addition of FDCs with the ICs gave a potent response. Here, we show a TI response without any factors beyond those provided by ICs and FDCs. Results showed that B cells stimulated with as low as 100 ng anti-IgD ICs loaded on FDCs produced IgM within ∼48 h in a B cell number-dependent fashion. In the absence of FDCs, anti-lgD ICs did not induce production of IgM even at doses of 10 µg/mL (data on right).

### DETAILED DESCRIPTION OF THE INVENTION

One of the primary embodiments of the present invention is methods for determining whether a particular agent, an antigen or a vaccine formulation might function in the production of protective immunity in a subject upon administration of the agent, antigen or vaccine formulation.

Many of the methods of the present invention use *in vitro* germinal center (GC) lymphoid tissue equivalents (LTEs). As used herein, GC LTEs are comprised of a co-culture of B cells and follicular dendritic cells (FDCs) or FDC-like cells (Heinemann & Peters (2005) BMC Immunol. 6, 23; WO 2005 / 118779; EP 04012622.9). In addition to B cells and FDCs, the GC LTEs of the present invention may comprises T cells. In preferred embodiments, all the cells are human cells.

GC LTEs are described, for example, in US 2007/0218054 (WO 07/075979), which discloses the incorporation of GCs into three-dimensional (3D) engineered tissue constructs (ETCs). The preparation of GC LTEs is described in the Examples of US 2007/0218054. ln an embodiment of the invention described therein, the GC was incorporated in the design of an artificial immune system (AIS) to examine immune (especially humoral) responses to vaccines and other agents. In a further embodiment of that invention, development of an *in vitro* GC added functionality to an AIS, in that it enabled generation of an *in vitro* human humoral response by human B lymphocytes that is accurate and reproducible, without using human subjects. The invention also enabled the evaluation of, for example, vaccines, allergens, and immunogens and activation of human B cells specific for a given antigen, which can then be used to generate antibodies. Embodiments of that invention comprised placing follicular dendritic cells (FDCs) in an ETC, such as a collagen cushion, microcarriers, inverted colloid crystal matrices, or other synthetic or natural extracellular matrix material, where they could develop in three dimensions. FDCs in the *in vivo* environment were attached to collagen fibers and did not circulate, as most immune system cells do. Thus, placing FDCs in, for example, a collagen matrix ought to be more *in vivo*-like.

FDCs are localized to the lymph follicles and they assist in B cell maturation by the presentation of intact antigen to the B cells. Such presentation occurs in the germinal centers of peripheral lymphoid organs and also results in class switching and B cell proliferation. FDCs present antigens to B cells in the form of an immune complex (IC), which is comprised of antigens and antibodies bound thereto. *In vivo,* immunogens are quickly converted into immune complexes (ICs) by antibodies persisting in immune animals from prior immunization(s) and ICs form in primary responses as soon as the first antibody is produced. These ICs are trapped by FDCs and this leads to GC formation. Immune complexes are typically poorly immunogenic *in vitro,* yet minimal amounts of antigen (converted into ICs *in vivo*) provoke potent recall responses.

FDCs render ICs highly immunogenic. In fact, in the presence of FDCs, ICs are more immunogenic than free antigen (Tew *et al.* (2001) *Trends Immunol.* **22***,* 361-367). A high density of FcyRllB on FDCs bind lg-Fc in the lC and consequently the lTlM (immunoreceptor tyrosine-based inhibitory motif) signal delivered via B cell-FcγRIIB may be blocked. Antigen-antibody complexes cross-linking BCRs initiate this inhibitory signal and FcyRllB on B cells. BCR is not cross-linked with B cell FcyRllB in the model and thus a high concentration of FcyRllB on FDCs minimizes the negative signal to the B cell. In addition, FDCs provide lC-coated bodies (iccosomes), which B cells find highly palatable. The iccosome membrane is derived from FDC membranes that have antigen, CD21 L, and Ig-Fc attached. Iccosomes bind tightly to B cells and are rapidly endocytosed (Szakal et al. (1988) J. Immunol. 140, 341-353). Binding of BCR and CD21 of the B cell to the iccosomal antigen-CD21 L-lg-Fc complex is likely important in the endocytosis process. The B cells process this FDC-derived antigen, present it, and thus obtain T cell help (Kosco et al. (1988) J. Immunol. 140, 354-360). Thus, these ligand-receptor interactions help stimulate B cells and provide assistance beyond that provided by T cells.

ICs trapped by FDCs lead to GC formation. GC formation is involved in the production of memory B cells, somatic hypermutation, selection of somatically mutated B cells with high affinity receptors, affinity maturation, and regulation of serum IgG with high affinity antibodies (Tew et al. (1990) Immunol. Rev. 117, 185-211; Berek & Ziegner (1993) Immunol. Today 14, 400-404; MacLennan & Gray (1986) Immunol. Rev. 91, 61-85; Kraal et al. (1982) Nature 298, 377-379; Liu et al. (1996) Immunity 4, 241-250; Tsiagbe et al. (1992) Immunol. Rev. 126, 113-141).

The GC is generally recognized as a center for production of memory B cells; we have found that cells of the plasmacytic series are also produced (Kosco et al. (1989) Immunol. 68, 312-318; DiLosa et al. (1991) J. Immunol. 1460, 4071-4077; Tew et al. (1992) Immunol. Rev. 126, 1-14). The number of antibody-forming cells (AFCs) in GCs peaks during an early phase (about 3 to about 5 days after secondary antigen challenge) and then declines. By about day 10 when GCs reach maximal size, there are very few AFCs present (Kosco et al. (1989) Immunol. 68, 312-318). During the early phase, GC B cells receive signals needed to become AFCs. The GC becomes edematous and the AFCs leave and we find them in the thoracic duct lymph and in the blood. These GC AFCs home to bone marrow where they mature and produce the vast majority of serum antibody (DiLosa et al. (1991) J. Immunol. 1460, 4071-4077; Tew et al. (1992) Immunol. Rev. 126, 1-14; Benner et al. (1981) Clin. Exp. Immunol. 46, 1-8). In the second phase, which peaks about 10-14 days after challenge, GCs enlarge, and the memory B cell pool is restored and expanded. Thus, production of B memory and fully functional and mature antibody responses appears to require GCs and FDCs.

Potentiating B cell viability can be done with or without FDCs present to enhance *in vitro* GC efficacy. A method is to add fibroblasts or other stromal cells, such as synovial tissue-derived stromal cell lines, the effects of which are to prolong B cell viability *in vitro* through cell-cell co-stimulation (e.g., Hayashida et al. (2000) J. Immunol, 164, 1110-1116). Another soluble agent that has been shown to increase naïve and memory B cell viability is reduced glutathione (GSH), perhaps through anti-oxidant activity (see Jeong et al. (2004) Mol. Cells 17, 430-437). Although Jeong et al. did not see enhanced viability of GC B cells, they did significantly enhance naïve and memory B cells with fibroblasts and GSH, suggesting that peripheral B lymphocytes can be used to populate the in vitro GC. Other soluble factors, such as IL-4, CD40L and anti-CD40 have been shown to potentiate B cell viability (L. Mosquera's work and M. Grdisa (2003) Leuk. Res. 27, 951-956). Ancillary factors and cells that increase B cell viability with or without FDCs will enhance in vitro GC performance.

*In vivo* FDCs exist in networks linked to collagen and collagen associated molecules. This linkage allows networks of FDCs to remain stationary while B cells and T cells move in and out of contact with the FDCs and associated antigen. This arrangement has been reconstructed in the *in vitro* GCs of the present invention.

*Vaccination Site Model.* Dendritic cells (DCs) are among the most potent antigen-presenting cells (APCs) and are the only known cell type with the capacity to stimulate naïve T cells in a primary immune response. Peripheral blood monocytes are widely accepted as a reliable source of precursor cells for DC generation *in vitro.* Such monocyte-derived DCs (mo-DCs) posses the overall phenotype and antigen-presenting abilities found in DCs *in vivo.*

A common generation technique for mo-DCs is based on using the cytokines GM-CSF and IL-4 for 5 days, leading to cells with an immature phenotype. After antigen priming for a subsequent 2 days, mo-DCs increase their co-stimulatory and antigen-presenting capabilities to a state called maturation.

Interestingly, Randolph et al. (1998) (Science 282, 480-3) found that the likely naturally occurring process of monocyte transendothelial migration induces a process of differentiation into DCs in just 2 days, without addition of exogenous cytokines. This process starts with monocytes traversing a monolayer of endothelial cells in the luminal to abluminal direction, followed by a reverse transmigration to the luminal surface after a period of 48 h of resting (interaction) within the extracellular matrix (susceptible of containing specific antigens).

In 1968, Szakal and Hanna (J. Immunol. 101, 949-962; Exp. Mol. Pathol. 8, 75-89) and Nossal et al. (J. Exp. Med. 127, 277-290) published the first descriptions and electron micrographs of what are now known as follicular dendritic cells (FDCs). Both groups used ¹²⁵I-labeled antigens and examined autoradiographs of the follicles in rodent spleens or lymph nodes using electron microscopy. Both groups found that radiolabel persisted on or near the surface of highly convoluted fine cell processes of dendritic-type cells with peculiar, irregularly shaped, euchromatic nuclei. The fine cell processes formed an elaborate meshwork around passing lymphocytes, allowing extensive cell-cell contact. Several names have been used for these cells but a nomenclature committee recommended the name "follicular dendritic cell" and the abbreviation "FDC" and these have been generally adopted (Tew et al. (1982) J. Reticuloendothelial Soc. 31, 371-380).

The ability of FDCs to trap and retain antigen-antibody complexes, together with their follicular location, distinguishes them from other cells, including other dendritic cells (DCs). FDCs bearing specific antigens are required for full development of GCs (Kosco et al. (1992) J. Immunol. 148, 2331-2339; Tew et al. (1990) Immunol. Rev. 117, 185-211) and are believed to be involved in lg class switching, production of B memory cells, selection of somatically mutated B cells with high affinity receptors, affinity maturation, induction of secondary antibody responses, and regulation of serum IgG with high affinity antibodies (Tew et al. (1990) Immunol. Rev. 117, 185-211; Berek & Ziegner (1993) Immunol. Today 14, 400-404; MacLennan & Gray (1986) Immunol. Rev. 91, 61-85; Kraal et al. (1982) Nature 298, 377-379; Liu et al. (1996) Immunity 4, 241-250; Tsiagbe et al. (1992) Immunol. Rev. 126, 113-141). Many researchers have worked with FDCs in culture in 2D with the general idea of mimicking an *in vivo* GC. An appreciation of the accessory functions of FDCs and regulation of these functions is important to an understanding of fully functional and mature antibody responses.

FDC development is B cell-dependent; FDCs are not detectable in, for example, SCID mice, mice treated with anti-mu (to remove B cells), or mice lacking the mu chain (where B cells do not develop) (MacLennan & Gray (1986) Immunol. Rev. 91, 61-85; Kapasi et al. (1993) J. Immunol. 150, 2648-2658). In T cell-deficient mice (e.g., nude mice), FDCs do develop, although the development is retarded and the FDCs do not appear to express many FDC markers (Tew et al. (1979) Aust. J. Exp. Biol. Med. Sci. 57, 401-414).

Reconstitution of FDCs in SCID mice occurs best when both B cells and T cells are adoptively transplanted, suggesting that T cells are also involved in FDC development (Kapasi et al. (1993) J. Immunol. 150, 2648-2658). Disruption of LT/TNF or the cognate receptors disrupts lymph node organogenesis and interferes with the development of FDC networks (De Togni et al. (1994) Science 264, 703-707; Rennert et al. (1996) J. Exp. Med. 184, 1999-2006; Chaplin & Fu (1998) Curr. Opin. Immunol. 10, 289-297; Endres et al. (1999) J. Exp. Med. 189, 159-168; Ansel et al. (2000) Nature 406, 309-314). As summarized by Debard et *al.*, it is known that a lack of LTαβ, LTβ, TNFαR1, and LTβR interferes with the development of FDC networks (19). B cells are an important source of LTα/β heterotrimers, consistent with data indicating that FDC development is B cell-dependent (Endres et al. (1999) J. Exp. Med. 189, 159-168; Ansel et al. (2000) Nature 406, 309-314; Fu et al. (1998) J. Exp. Med. 187, 1009-1018).

The functional element of a mammalian lymph node is the follicle, which develops a GC when stimulated by an antigen. The GC is an active area in a lymph node, where important interactions occur in the development of an effective humoral immune response. Upon antigen stimulation, follicles are replicated and an active human lymph node may have dozens of active follicles, with functioning GCs. Interactions between B cells, T cells, and FDCs take place in GCs. Various studies of GCs *in vivo* indicate that the following events occur there:
- immunoglobulin (Ig) class switching,
- rapid B cell proliferation (GC dark zone),
- production of B memory cells,
- accumulation of select populations of antigen specific T cells and B cells,
- hypermutation,
- selection of somatically mutated B cells with high affinity receptors,
- apoptosis of low affinity B cells,
- affinity maturation,
- induction of secondary antibody responses, and
- regulation of serum immunoglobulin G (IgG) with high affinity antibodies.

Similarly, data from *in vitro* GC models indicate that FDCs are involved in:
- stimulating B cell proliferation with mitogens and it can also be demonstrated with antigen (Ag),
- promoting production of antibodies including recall antibody responses,
- producing chemokines that attract B cells and certain populations of T cells,
   and
- blocking apoptosis of B cells.

While T cells are necessary for B cell responses to T cell-dependent antigens, they are not sufficient for the development of fully functional and mature antibody responses that are required with most vaccines. FDCs provide important assistance needed for the B cells to achieve their full potential (Tew et al. (2001) Trends Immunol. 22, 361-367).

Humoral responses in vaccine assessment can be examined using an artificial immune system (AIS). Accessory functions of follicular dendritic cells and regulation of these functions are important to an understanding of fully functional and mature antibody responses.

Important molecules have been characterized by blocking ligands and receptors on FDCs or B cells. FDCs trap antigen-antibody complexes and provide intact antigen for interaction with B cell receptors (BCRs) on GC B cells; this antigen-BCR interaction provides a positive signal for B cell activation and differentiation. Engagement of CD21 in the B cell co-receptor complex by complement derived FDC-CD21 L delivers an important co-signal. Coligation of BCR and CD21 facilitates association of the two receptors and the cytoplasmic tail of CD19 is phosphorylated by a tyrosine kinase associated with the B cell receptor complex (Carter et al. (1997) J. Immunol. 158, 3062-3069). This co-signal dramatically augments stimulation delivered by engagement of BCR by antigen and blockade of FDC-CD21 L reduces the immune responses ∼10- to ∼1,000-fold.

### Test Agent

As used in the methods of the present invention, a test antigen is a molecule for which information regarding its ability to induce an immune response is desired. As further indicated herein, the ability of a test antigen to induce an immune response can be determined based on the ability of the test antigen to induce production of IgM or IgG using the methods described herein. The test antigens used in the methods of the present invention are limited only in that they can be administered to the GC LTEs of the present invention.

In a preferred embodiment the test agent is an antigen against which it is desired to induce an immune response in a subject (upon administration of the antigen in a vaccine formulation to a subject). Such antigens include polypeptides, peptides, proteins and polysaccharides. In preferred embodiments the test agents are proteins or polysaccharides derived from a bacteria or virus having the ability to infect and cause disease in a human. Thus, for example, test agents may be surface or integral membrane proteins of bacteria or coat proteins of viruses. The test agent may be an entire polypeptide or polysaccharide, or a portion of thereof. In one embodiment, the test agent may be the entire organism (e.g., bacteria virus) against which it is desired to raised an immune response. In this embodiment, preferably the organism is attenuated such that it can no longer cause disease or an infection in the subject to which it is administered.

In other embodiments the test agent may be a non-biological molecule, for which information regarding the molecules antigenicity is desired.

### Immune Complexes

An embodiment of the present invention concerns antigen-antibody complexes (immune complexes, ICs) that can be used, for example, in *in vitro* GC LTEs and which may be used, for example, for pre-clinically evaluating vaccine candidates and other immunomodulatory agents.

Immune complexes play an important role in the function of follicular dendritic cells (FDC), which are principally responsible for regulating the differentiation of antigen-specific B cells into high-affinity antibody producers in the generation of a humoral immune response. *In vitro* experiments have shown that B cells stimulated to produce antibody in the absence of IC-loaded FDC are not capable of fully differentiating into high-affinity antibody producers. Consequently, specific lC will be important in eliciting a humoral immune response within the AIS.

As used herein, an immune complex or lC comprises an antibody and an antigen to which it is bound. The skilled artisan will understand that there are no limitations on the identities of the antibodies and antigens that comprise the immune complexes of the present invention. For example, the antibodies may be obtained from any species of animal, though preferably from a mammal such as a human, simian, mouse, rat, rabbit, guinea pig, horse, cow, sheep, goat, pig, dog or cat. Preferably the antibodies are human antibodies. Nor is there a limitation on the particular class of antibody that may comprise the immune complex, including IgG1, lgG2, lgG3, IgG4, IgM, IgA1, lgA2, IgD and IgE antibodies. Antibody fragments of less than the entire antibody may also be used, including single chain antibodies, F(ab')₂ fragments, Fab fragments, and fragments produced by an Fab expression library, with the only limitation being that the antibody fragments retain the ability to bind the antigen.

The antibodies may also be polyclonal, monoclonal, or chimeric antibodies, such as where an antigen binding region (e.g., F(ab')₂ or hypervariable region) of a non-human antibody is transferred into the framework of a human antibody by recombinant DNA techniques to produce a substantially human molecule.

For the production of antibodies, various hosts including, but not limited to, goats, rabbits, rats, mice, humans, etc., can be immunized by injection with a particular protein or any portion, fragment, or oligopeptide that retains immunogenic properties of the protein. Depending on the host species, various adjuvants can be used to increase the immunological response. Such adjuvants include, but are not limited to, detoxified heat labile toxin from *E*. *coli,* Freund's, mineral gels such as aluminum hydroxide, and surface active substances such as lysolecithin, pluronic polyols, polyanions, peptides, oil emulsions, keyhole limpet hemocyanin, and dinitrophenol. BCG (*Bacillus Calmette-Guerin)* and *Corynebacterium parvum* are also potentially useful adjuvants.

Antibodies and fragments thereof can be prepared using any technique that provides for the production of antibody molecules, such as by continuous cell lines in culture for monoclonal antibody production. Such techniques include, but are not limited to, the hybridoma technique originally described by Koehler and Milstein (Nature 256:495-497 (1975)), the human B-cell hybridoma technique (Kosbor et al., Immunol Today 4:72 (1983); Cote et al., Proc Natl. Acad. Sci 80:2026-2030 (1983)), and the EBV-hybridoma technique (Cole et al., Monoclonal Antibodies and Cancer Therapy, Alan R. Liss Inc, New York N.Y., pp 77-96 (1985)).

Techniques developed for the production of "chimeric antibodies," i.e., the splicing of mouse antibody genes to human antibody genes to obtain a molecule with appropriate antigen specificity and biological activity, can also be used (Morrison et al., Proc Natl. Acad. Sci 81:6851-6855 (1984); Neuberger et al., Nature 312:604-608(1984); Takeda et al., Nature 314:452-454(1985)). Alternatively, techniques described for the production of single chain antibodies, such as disclosed in U.S. Patent No. 4,946,778, incorporated herein by reference in its entirety, can be adapted to produce Aap-specific single chain antibodies. Additionally, antibodies can be produced by inducing *in vivo* production in the lymphocyte population or by screening recombinant immunoglobulin libraries or panels of highly specific binding reagents as disclosed in Orlandi et al., Proc Natl. Acad. Sci. USA 86: 3833-3837 (1989); and Winter G. and Milstein C., Nature 349:293-299 (1991).

Antibody fragments such as F(ab')₂ fragments can be produced by pepsin digestion of the antibody molecule, and Fab fragments can be generated by reducing the disulfide bridges of the F(ab')₂ fragments. Alternatively, Fab expression libraries can be constructed to allow rapid and easy identification of monoclonal Fab fragments with the desired specificity. (Huse W. D. et al., Science 256:1275-1281 (1989)).

The antigens that comprise the immune complexes of the present invention are limited only in that they are bound by the antibody of the immune complex. Thus, in general terms, the antigen is a small molecule, such as a peptide of 10-15 amino acids. Generally the antigens comprising the immune complexes of the present invention will be a portion of a larger antigen that is present in the vaccine formulations of the present invention or a portion of a test agent of the present invention. For example, as explained further herein the vaccine formulations of the present invention include an antigen and a pharmaceutically acceptable carrier or diluent. Such antigens found in the vaccine formulations may be any antigen against which it is desired to induce an immune response in a subject (upon administration of the vaccine formulation to a subject). Such antigens include polypeptides, peptides, proteins and polysaccharides. The skilled artisan will thus understand that while the immune complexes of the present invention comprise an antibody and at least a portion of an antigen, to which the antibody is bound.

As indicated above, the development of ICs first requires the generation of antibodies reactive against the antigen of interest. Specific antibodies can be elicited by immunizing animals with antigen directly, but this can be a costly, slow, and inconvenient procedure. While it is also possible to generate reactive antibody by stimulating naïve B cells *in vitro,* this also can be a laborious technique that typically yields only small quantities of specific antibody.

An embodiment of the present invention thus comprises approaches to generating ICs by artificially coupling antibody to antigen in a non-specific manner. This offers the following advantages over existing techniques:
- pre-existing antibodies can be used,
- a large amount of lC can be produced without the need of generating a specific immune response,
- one antibody can be coupled to many different antigens, and
- lC development will be much quicker than is possible with current methods.

In an embodiment of the present invention, ICs can be generated by coupling a hapten to the antigen of interest, which can then be bound by a specific antibody. As an example, fluorescein isothiocyanate (FITC) of Fluorescein-EX dyes can be conjugated to primary amino groups on a target protein, using literature procedures In this regard, Fluorescein-EX or other derivatives bearing elongated linkers may be advantageous over tight linker-antigen conjugates formed by FITC and other haptens. Commercially available high-affinity anti-FITC antibodies can then be used to bind the antigen-hapten conjugate, forming a complete IC. Tetanus toxoid can be used as a model antigen, because most adults are immunized against it and the humoral and cell-mediated immune responses generated against this antigen are well known. In other embodiments, other linkers (e.g., digoxin) and antigens (e.g., ovalbumin) can be used. In another embodiment, the antibody can be chemically coupled to the antigen using, for example, the amine-thiol cross-linking method that is often used to form protein heteroconjugates. Using these non-specific chemistries does not require an agglutination step, making them useful for polyclonal antibodies. Additionally, the stoichiometry of the lC can be manipulated without affecting the size or density of this complex.

### Vaccine Formulations

As indicated above, the present invention is also directed to methods for utilizing and testing vaccine formulations. As used herein, a vaccine formulation comprises at least one antigen and a pharmaceutically acceptable carrier or diluent.

The antigens comprising the vaccine formulations of the present invention may be any antigen against which it is desired to induce an immune response in a subject (upon administration of the vaccine formulation to a subject) or for which information regarding its antigenicity is desired to be known. Such antigens include polypeptides, peptides, proteins and polysaccharides. In preferred embodiments the antigens comprising the vaccine formulations of the present invention are derived from a bacteria or virus having the ability to infect and cause disease in a human. Thus, for example, antigens comprising a vaccine formulation may include surface or integral membrane proteins of bacteria or coat proteins of viruses. The antigen may be an entire polypeptide or polysaccharide, or a portion of thereof. In one embodiment, the antigen may be the entire organism (e.g., bacteria virus) against which it is desired to raised an immune response. In this embodiment, preferably the organism is attenuated such that it can no longer cause disease or an infection in the subject to which it is administered.

The amount of the antigen present in the vaccine formulation will vary based on the identity of the antigen and will thus be determined by the skilled artisan. However, in certain methods of the present invention the amount of antigen in a vaccine formulation will typically be an amount sufficient to induce an immune response in a subject, preferably a protective immune response to the organism from which the antigen was derived.

The vaccine formulations used in the methods of the present invention will preferably be in a formulation that is similar to or identical to the formulation that would be administered to a subject. However, the skilled artisan will also understand that the methods of the present invention may utilize a vaccine formulation comprising at least one antigen and an inert carrier or diluent, such as water or buffered solution.

### Two-Component Vaccine Systems

The present invention is also directed to a two-component vaccine system and to methods for utilizing and testing a two-component vaccine system. As used herein, a two-component vaccine system comprises two components, wherein the first component comprises at least one antigen, preferably in a pharmaceutically acceptable carrier or diluent, and wherein the second component comprises an immune complex comprising the antigen of the first component and an antibody bound thereto, preferably in a pharmaceutically acceptable carrier or diluent.

The antigens comprising the two-component vaccine systems of the present invention may be any antigen against which it is desired to induce an immune response in a subject (upon administration of the vaccine formulation to a subject) or for which information regarding its antigenicity is desired to be known. Such antigens include polypeptides, peptides, proteins and polysaccharides. In preferred embodiments the antigens comprising the two-component vaccine systems of the present invention are derived from a bacteria or virus having the ability to infect and cause disease in a human. Thus, for example, antigens comprising a two-component vaccine system may include surface or integral membrane proteins of bacteria or coat proteins of viruses. The antigen may be an entire polypeptide or polysaccharide, or a portion of thereof. In one embodiment, the antigen may be the entire organism (e.g., bacteria virus) against which it is desired to raised an immune response. In this embodiment, preferably the organism is attenuated such that it can no longer cause disease or an infection in the subject to which it is administered.

The amount of the antigen present in the two-component vaccine system will vary based on the identity of the antigen and will thus be determined by the skilled artisan. However, in certain methods of the present invention the amount of antigen in a two-component vaccine system will typically be an amount sufficient to induce an immune response in a subject, preferably a protective immune response, to the organism from which the antigen was derived.

Each of the components of the two-component vaccine systems of the present invention will preferably be in a formulation that is similar to or identical to the formulation that would be administered to a subject. However, the skilled artisan will also understand that the methods of the present invention may utilize a two-component vaccine system wherein each component comprises an inert carrier or diluent, such as water or buffered solution.

In a preferred embodiment each of the components of the two-component vaccine systems of the present invention are separately formulated and in separate containers. However, it is envisioned that in certain embodiments the two components could be mixed in the same container.

In a preferred embodiment of the methods directed to inducing an immune response in a subject using the two-component vaccine system of the present invention, the two components are administered to separate sites of a subject. Thus, when the components are administered via an injection, the injection sites are different locations, for example, the left arm and the right arm of an animal, such as a human, or the left leg and right of an animal, such as a human. The components may be administered at the same time, or sequentially. In a preferred embodiment the components are administered within less than 15 minutes, 30 minutes, 45 minutes, one hour, two hours, three hours, four hours, five hours or more, of each other.

As indicated above, each of the components in the two-component vaccine system may be formulated with a pharmaceutically acceptable carrier or diluent. As such each of the components in the two-component vaccine system can be formulated in a variety of useful formats for administration by a variety of routes. Administration of the components of the two-component vaccine system can be by any means generally used in the art, and includes intravenous, intraperitoneal, intramuscular, subcutaneous and intradermal routes, nasal application, by inhalation, ophthalmically, orally, rectally, vaginally, or by other means that results in the vaccine components contacting mucosal tissues.

Injectable formulations of the components of the two-component vaccine system for administration via intravenous, intraperitoneal, intramuscular, subcutaneous and intradermal routes may include various carriers such as vegetable oils, dimethylacetamide, dimethylformaamide, ethyl lactate, ethyl carbonate, isopropyl myristate, ethanol, polyols (glycerol, propylene glycol, and liquid polyethylene glycol) and the like. Intramuscular preparations can be prepared and administered in a pharmaceutical excipient such as Water-for-Injection, 0.9% saline, or 5% glucose solution.

Solid formulations for oral administration may contain suitable carriers or diluents, such as corn starch, gelatin, lactose, acacia, sucrose, microcrystalline cellulose, kaolin, mannitol, dicalcium phosphate, calcium carbonate, sodium chloride, or alginic acid. Disintegrators that can be used include, without limitation, microcrystalline cellulose, cornstarch, sodium starch glycolate, and alginic acid. Tablet binders that may be used include acacia, methylcellulose, sodium carboxymethylcellulose, polyvinylpyrrolidone, hydroxypropyl methylcellulose, sucrose, starch, and ethylcellulose. Lubricants that may be used include magnesium stearates, stearic acid, ailicone fluid, talc, waxes, oil, and colloidal silica.

In one embodiment of the present invention, each of the components in the two-component vaccine system may exist as atomized dispersions for delivery by inhalation. The atomized dispersion typically contains carriers common for atomized or aerosolized dispersions, such as buffered saline and/or other compounds well known to those of skill in the art. The delivery of the components via inhalation has the effect of rapidly dispersing the vaccine components to a large area of mucosal tissues as well as quick absorption by the blood for circulation. One example of a method of preparing an atomized dispersion is described in U.S. Patent No. 6,187,344, entitled, "Powdered Pharmaceutical Formulations Having Improved Dispersibility," which is hereby incorporated by reference in its entirety.

The components in the two-component vaccine system described herein can also be formulated in the form of a rectal or vaginal suppository. Typical carriers used in the formulation of the inactive portion of the suppository include polyethylene glycol, glycerine, cocoa butter, and/or other compounds well known to those of skill in the art.

Additionally, the components in the two-component vaccine system may be administered in a liquid form. The liquid can be for oral dosage, for ophthalmic or nasal dosage as drops, or for use as an enema or douche. When the vaccine components are formulated as a liquid, the liquid can be either a solution or a suspension of the vaccine components. There are a variety of suitable formulations for the solution or suspension of the vaccine components that are well know to those of skill in the art, depending on the intended use thereof. Liquid formulations for oral administration prepared in water or other aqueous vehicles may contain various suspending agents such as methylcellulose, alginates, tragacanth, pectin, kelgin, carrageenan, acacia, polyvinylpyrrolidone, and polyvinyl alcohol. The liquid formulations may also include solutions, emulsions, syrups and elixirs containing, together with the active compound(s), wetting agents, sweeteners, and coloring and flavoring agents. Various liquid and powder formulations can be prepared by conventional methods for inhalation into the lungs of the mammal to be treated.

Each of the components of the two-component vaccine system of the present invention may be administered in a single dose or in multiple doses over prolonged periods of time, such as up to about one week, and even for extended periods longer than one month or one year. In some instances, administration of the components may be discontinued and resumed at a later time. For example, a second dose can be administered 28 days later, or at some other time interval to be determined. Serum for antibody assessment can be collected prior to immunization and fourteen days following each dose. Sera is then assessed for antibodies against the antigen in the vaccine system.

A kit comprising the necessary components of the two-component vaccine system for inducing an immune response in a subject and instructions for their use are also within the purview of the present invention.

### Measurina Antibody/Antiaen Interactions

Antibody affinity is the strength of the reaction between a single antigenic determinant and a single combining site on an antibody. It is the sum of the attractive and repulsive forces operating between the antigenic determinant and the combining site of the antibody. Most antibodies have a high affinity for their antigens. Avidity is a measure of the overall strength of binding of an antigen with many antigenic determinants and multivalent antibodies. Avidity is influenced by both the valence of the antibody and the valence of the antigen. Avidity is more than the sum of the individual affinities.

Antibody affinity can be assessed, for example, by determining the equilibrium K_{D}, which can be estimated for moderate to high affinity interactions using a series of antibody/antigen concentrations (see, *e.g.,* Daugherty et al. (1998) Protein Engineering 11, 101-108 and Nolan & Sklar (1998) Nature Biotechnol. 16, 633-8).

The ease with which one can detect antigen-antibody reactions will depend on a number of factors, including affinity (the higher the affinity of the antibody for the antigen, the more stable will be the interaction), avidity (reactions between multivalent antigens and multivalent antibodies are more stable and thus easier to detect), the antigen to antibody ratio (the ratio between the antigen and antibody influences the detection of antigen-antibody complexes because the size of the complexes formed is related to the concentration of the antigen and antibody), and the physical form of the antigen (e.g., if the antigen is a particulate, generally, agglutination of the antigen by the antibody is used, whereas if the antigen is soluble, generally, the precipitation of the antigen after the production of large insoluble antigen-antibody complexes is used).

When an antigen is particulate, the reaction of an antibody with the antigen can be detected, for example, by agglutination (clumping) of the antigen. The general term agglutinin is used to describe antibodies that agglutinate particulate antigens. All antibodies can theoretically agglutinate particulate antigens but IgM, due to its high valence, is a particularly good agglutinin and it can sometimes be inferred that an antibody may be of the IgM class if it is a good agglutinating antibody.

Agglutination tests can be used in a qualitative manner to assay for the presence of an antigen or an antibody. The antibody is mixed with the particulate antigen and a positive test is indicated by the agglutination of the particulate antigen. Agglutination tests can also be used to measure the level of antibodies to particulate antigens. In this test, serial dilutions are made of a sample to be tested for antibody and then a fixed number of red blood cells or bacteria or other such particulate antigen is added. Then the maximum dilution that gives agglutination is determined. The maximum dilution that gives visible agglutination is called the titer. The results are reported as the reciprocal of the maximal dilution that gives visible agglutination.

*Passive hemagglutination -* The agglutination test only works with particulate antigens. However, it is possible to coat erythrocytes with a soluble antigen (e.g. viral antigen, a polysaccharide or a hapten) and use the coated red blood cells in an agglutination test for antibody to the soluble antigen, referred to as passive hemagglutination. The test is performed just like the agglutination test. Applications include detection of antibodies to soluble antigens and detection of antibodies to viral antigens.

If the antigen is soluble, generally, the precipitation of the antigen after the production of large insoluble antigen-antibody complexes is used. Such precipitation tests include the radial immunodiffusion assay of Mancini (Mancini et al. (1965) Immunochemistry 2, 235-54*;* Mancini et al. (1970) Immunochemistry 7, 261-4). In radial immunodiffusion, antibody is incorporated into an agar gel as it is poured and different dilutions of the antigen are placed in holes punched into the agar. As the antigen diffuses into the gel, it reacts with the antibody and when the equivalence point is reached a ring of precipitation is formed. The diameter of the ring is proportional to the log of the concentration of antigen because the amount of antibody is constant. Thus, by running different concentrations of a standard antigen a standard curve is prepared, from which the amount of an antigen in an unknown sample can be quantitated; thus, it is a quantitative test. If more than one ring appears in the test, more than one antigen/antibody reaction has occurred. This could be due to a mixture of antigens or antibodies. This test is commonly used in the clinical laboratory for the determination of immunoglobulin levels in patient samples.

Another technique is that of immunoelectrophoresis. In immunoelectrophoresis, a complex mixture of antigens is placed in a well punched out of an agar gel and the antigens are electrophoresed so that the antigens are separated according to charge. After electrophoresis, a trough is cut in the gel and antibodies are added. As the antibodies diffuse into the agar, precipitin lines are produced in the equivalence zone when an antigen/antibody reaction occurs. This test is used for the qualitative analysis of complex mixtures of antigens, although a crude measure of quantity (thickness of the line) can be obtained. This test is commonly used for the analysis of components in a patient's serum. Serum is placed in the well and antibody to whole serum in the trough. By comparisons to normal serum, one can determine whether there are deficiencies on one or more serum components or whether there is an overabundance of some serum component (thickness of the line).

Radioimmunoassays (RIA) are assays based on the measurement of radioactivity associated with immune complexes. In any particular test, the label may be on either the antigen or the antibody.

Enzyme-linked immunosorbent assays (ELISAa) are based on the measurement of an enzymatic reaction associated with immune complexes. In any particular assay, the enzyme may be linked to either the antigen or the antibody.

Specific determination of, for example, mouse, rabbit or human IgG or IgM concentrations can be made with commercially available reagents, such as the Easy-Titer Antibody Assay Kits (Pierce), or by ELISA. The Easy-Titer Antibody Assay Kits include antibody-sensitized microspheres to measure the specific concentration of mouse, rabbit and human antibodies by an easy and rapid microagglutination technique using standard microplates and UV-Vis plate reader (spectrophotometer). Each kit is specific for a particular species and class of immunoglobulin and, unlike total protein assays, can specifically measure the concentration of target antibody in samples (e.g., serum, plasma, culture supernatant) that contain other proteins. The kits are sensitive, requiring very small sample volumes. Antibody concentration is determined from the assay response (absorbance) by comparison to a standard curve prepared using dilutions of a known antibody sample. Easy-Titer Assay Kits detect and measure specific target antibodies using agglutination of microspheres that are coated ("sensitized") with the specific anti-lgG or IgM polyclonal antibodies.

Particular embodiments of the invention are:
1. A method for determining whether a test agent is antigenic, comprising:
   (a) contacting an *in vitro* germinal center (GC) lymphoid tissue equivalent (LTE) with a test agent under conditions promoting production of IgM, wherein the *in vitro* GC LTE comprises:
      (i) B cells, and
      (ii) follicular dendritic cells (FDCs) or FDC-like cells, wherein the follicular dendritic cells (FDCs) or FDC-like cells are loaded with immune complexes (ICs) comprising at least a portion of the test agent; and
   (b) assaying the *in vitro* GC LTE of (a) for IgM production,
      wherein when production of agent-specific IgM is found in (b), the test agent is determined to be antigenic.
2. The method of embodiment 1 wherein the test agent is selected from the group consisting of a peptide, a polypeptide, a protein, and a polysaccharide.
3. A method for determining whether a vaccine formulation is antigenic, comprising:
   (a) contacting an *in vitro* germinal center (GC) lymphoid tissue equivalent (LTE) with a vaccine formulation under conditions promoting production of IgM, wherein the vaccine formulation comprises at least one antigen and wherein the *in vitro* GC LTE comprises:
      (i) B cells, and
      (ii) follicular dendritic cells (FDCs) or FDC-like cells, wherein the follicular dendritic cells (FDCs) or FDC-like cells are loaded with immune complexes (ICs) comprising at least a portion of the antigen comprising the vaccine formulation; and
   (b) assaying the *in vitro* GC LTE of (a) for IgM production,
      wherein when production of antigen-specific IgM is found in (b), the vaccine formulation is determined to be antigenic.
4. A method for determining the antigenicity of a vaccine formulation, comprising:
   (a) contacting an *in vitro* germinal center (GC) lymphoid tissue equivalent (LTE) with a vaccine formulation under conditions promoting production of IgM, wherein the vaccine formulation comprises at least one antigen and wherein the *in vitro* GC LTE comprises:
      (i) B cells, and
      (ii) follicular dendritic cells (FDCs) or FDC-like cells, wherein the follicular dendritic cells (FDCs) or FDC-like cells are loaded with immune complexes (ICs) comprising at least a portion of the antigen comprising the vaccine formulation; and
   (b) determining the amount of antigen-specific IgM produced by the *in vitro* GC LTE of (a), wherein the amount of antigen-specific IgM determined in (b) corresponds to the antigenicity of the vaccine formulation, thereby determining the antigenicity of a vaccine formulation.
5. A method for determining the antigenicity of a vaccine formulation, comprising:
   (a) contacting an *in vitro* germinal center (GC) lymphoid tissue equivalent (LTE) with a vaccine formulation under conditions promoting production of IgM, wherein the vaccine formulation comprises at least one antigen and wherein the *in vitro* GC LTE comprises:
      (i) B cells, and
      (ii) follicular dendritic cells (FDCs) or FDC-like cells, wherein the follicular dendritic cells (FDCs) or FDC-like cells are loaded with immune complexes (ICs) comprising at least a portion of the antigen comprising the vaccine formulation; and
   (b) collecting antigen-specific IgM produced by the *in vitro* GC LTE of (a);
      and
   (c) determining the affinity of the antigen-specific IgM collected in (b) for the antigen, wherein the affinity of the antigen-specific IgM determined in (c) for the antigen corresponds to the antigenicity of the vaccine formulation, thereby determining the antigenicity of a vaccine formulation.
6. A method for determining whether a two-component vaccine system is antigenic, comprising:
   (a) contacting an *in vitro* germinal center (GC) lymphoid tissue equivalent (LTE) with a first component of a two-component vaccine system under conditions promoting production of IgM, wherein the first component of the two-component vaccine system comprises an antigen and wherein the *in vitro* GC LTE comprises:
      (i) B cells, and
      (ii) follicular dendritic cells (FDCs) or FDC-like cells, wherein the follicular dendritic cells (FDCs) or FDC-like cells are loaded with immune complexes (ICs) comprising at least a portion of the antigen comprising the first component of the two-component vaccine system;
   (b) contacting the *in vitro* GC LTE of (a) with a second component of the two-component vaccine system under conditions promoting production of IgM, wherein the second component of the two-component vaccine system comprises the antibody and the portion of the antigen of the ICs of (a); and
   (c) assaying the *in vitro* GC LTE of (b) for IgM production,
      wherein when production of antigen-specific IgM is found in (c), the vaccine is determined to be antigenic.
7. A method for generating IgM antibodies, comprising:
   (a) contacting an *in vitro* germinal center (GC) lymphoid tissue equivalent (LTE) with an antigen, wherein the *in vitro* GC LTE comprises:
      (i) B cells, and
      (ii) follicular dendritic cells (FDCs) or FDC-like cells, wherein the follicular dendritic cells (FDCs) or FDC-like cells are loaded with immune complexes (ICs) comprising at least a portion of the antigen; and
   (b) culturing the *in vitro* GC LTE of (a) under conditions promoting generating of IgM antibodies, thereby generating IgM antibodies.
8. The method of embodiment 7 wherein the culturing (b) is for about 48 hours.
9. The method of embodiment 7 wherein the culturing (b) is for about 72 hours.
10. The method of embodiment 7 further comprising collecting IgM antibodies generated in (b).
11. The method of embodiment 7 further comprising culturing (b) until antibody class switching is achieved.
12. The method of embodiment 11, wherein the class switching is switching from IgM production to IgG production.
13. The method of embodiment 1, wherein the B cells of the *in vitro* GC LTE are exposed to the test agent prior to contacting of the *in vitro* GC LTE with the test agent.
14. The method of embodiment 3, 4 or 5, wherein the B cells of the *in vitro* GC LTE are exposed to the antigen prior to contacting of the *in vitro* GC LTE with the vaccine.
15. The method of embodiment 6, wherein the B cells of the *in vitro* GC LTE are exposed to the first component of the two-component vaccine system prior to contacting of the *in vitro* GC LTE with first component of the two-component vaccine system.
16. The method of embodiment 6, wherein the B cells of the *in vitro* GC LTE are exposed to the second component of the two-component vaccine system prior to contacting of the *in vitro* GC LTE with first component of the two-component vaccine system.
17. The method of embodiment 6 wherein the antibody of the second component binds the portion of the antigen of the ICs of (a).
18. A two-component vaccine system comprising a first component and a second component, wherein the first component comprises an antigen and wherein the second component comprises an immune complex of the antigen of the first component.
19. The two-component vaccine system of embodiment 18 wherein the first component further comprises a pharmaceutically acceptable carrier or diluent and the second component further comprises a pharmaceutically acceptable carrier or diluent.
20. A method of inducing an immune response in a subject comprising
   (a) administering a first component of a two-component vaccine system to a subject, wherein the first component comprises an antigen and pharmaceutically acceptable carrier or diluent, and
   (b) administering a second component of the two-component vaccine system to the subject, wherein the second component comprises an immune complex of the antigen of the first component and pharmaceutically acceptable carrier or diluent.
21. The method of embodiment 20 wherein the second component of the two-component vaccine system is administered to a different location of the subject than the first component of the two-component vaccine system.
22. The method of embodiment 20 wherein the first and second components of the two- component vaccine system are administered concurrently or sequentially to the subject.
23. The method of embodiment 21 wherein the first and second components of the two- component vaccine system are administered concurrently or sequentially to different locations of the subject.
24. The method of embodiment 20 wherein the immune response is a rapid production of high-affinity antibodies.
25. The method of embodiment 24 wherein the high-affinity antibodies are high-affinity IgM antibodies or high-affinity IgG antibodies.
26. The method of embodiment 24 wherein the high-affinity antibodies are produced within about 24 hours after administration of the two-component vaccine system.
27. The method of embodiment 20 wherein the immune response is a protective immune response.

### Examples

### Example 1. FDC-like cells function like FDCs

FDC-like cells can be derived from human monocytes using published techniques (Heinemann & Peters (2005) BMC Immunol. 6, 23; see also WO 2005 / 118779 and EP 04012622.9. Use of these FDC-like cells is an advantage over isolating FDCs from human tonsils, which are not always readily available. An alternative is isolating FDCs from secondary lymphoid tissues of animals, but isolating functionally active FDCs from secondary lymphoid tissue requires considerable skill and there are times when introducing animal cells into a human system is not acceptable. Thus, it is desirable to be able to use readily available human FDC-like cells that have accessory activity comparable with FDCs.

We examined whether FDC-like cells could trap ICs like FDCs. To test this, FDCs and FDC-like cells were incubated with labeled ICs, the cells were washed to remove unbound ICs, and incubated overnight (∼15 h). Phagocytic cells can trap ICs, but such ICs will be endocytosed and destroyed during the overnight incubation. In contrast, FDCs trap ICs on their surfaces and the ICs *persist* on the cell surface for many months to years *in vivo.* Both FDCs and FDC-like cells trapped and retained ICs after overnight incubation (data not shown).

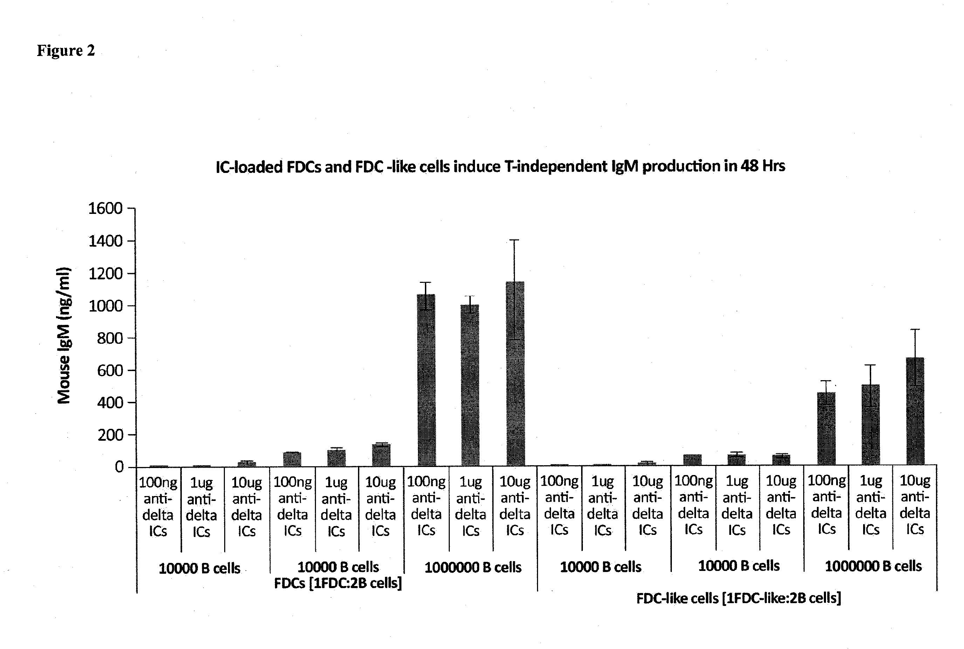

We next examined whether lC-bearing FDC-like cells had accessory activity and could promote the production of antibodies. To test this, FDCs and FDC-like cells were loaded with rat anti-mouse IgD, complexed with specific anti-rat IgG. We reasoned that the anti-delta in the ICs would bind membrane IgD on the B cells and provide a potent signal, causing the B cells to begin making IgM. When FDCs or FDC-like cells were loaded with ICs and incubated with purified B cells, they rapidly formed similar clusters with B cells that were typical of GC reactions *in vitro.* After ∼48 h the supernatant fluids were collected and the IgM response was determined. The results are illustrated in Figure 2.

FDCs are on the left side and FDC-like cells are on the right, at a ratio of 1 FDC or FDC-like cell to 2 B cells. A high number of FDC-like cells was chosen to ensure that we would see accessory activity, even if it was weaker in FDC-like cells than in FDCs. IgD ICs were used over a range, from ∼100 ng to ∼10 µg. However, ∼100 ng appeared to be adequate, as there were not a significant increase in Ab production with higher levels of lC. The B cells were used in 10-fold increases, from ∼10000, ∼100,000 to ∼10⁶ B cells. The Ab response followed with the 10-fold increases in B cells corresponding increases in IgM production (from ∼10 ng/mL, to ∼100 ng/mL, to ∼1000 ng/mL at the highest dose of B cells). It was observed that the FDC-like cells gave similar results, from ∼5 ng/mL, to 50 ng/mL, to 500 ng/mL, at the highest dose of B cells. In short, the patterns were very similar with the two cell types, there being about a two-fold increase in Ab production in favor of the FDCs, under these experimental conditions.

### Example 2. Rapid in vitro antigenicity assessment

Using FDCs or FDC-like cells, the *in vitro* GC LTE of the present invention can be used to rapidly assess the antigenicity of antigens. FDCs or FDC-like cells loaded with lC can be used to induce a rapid (∼48 h) IgM response. The B cell repertoire can be assessed, as can the antigenicity of the antigens.

### Example 3. Rapid in vitro vaccine assessment

Using FDCs or FDC-like cells, the *in vitro* GC LTE of the present invention can be used to rapidly assess the antigenicity of vaccine candidates. FDCs or FDC-like cells loaded with lC can be used to induce a rapid (∼48 h) IgM response. The B cell repertoire can be assessed, as can the antigenicity of the antigens.

### Example 4. Using the enhancing effect of ICs

A problem with the use of ICs is that they do not always activate DCs and prime T cells. In an embodiment of the present invention, a dual immunization strategy is used, in which ICs are targeted to FDCs to initiate an early IgM response and expand the specific B cells. Free antigen is then also injected into a different site to target DCs for T cell priming. With this dual immunization strategy, rapid specific IgM responses and enhanced IgG responses are induced. As an example, we looked at what happened when T cell help is provided with the ICs. This should bypass DCs and the need for T cell priming.

### Example 5. Dual immunization

Activating and inhibitory FcyRs appear to regulate signaling in DCs. For example, selective blockade of inhibitory FcγRIIB enables human dendritic cell maturation (Dhodapkar et al. (2005) Proc. Natl. Acad. Sci. USA 102, 2910-2915).

Accordingly, to avoid FcyRllB on DCs, free antigen was used rather than ICs while priming T cells for the *in vitro* primary in our germinal center (GC) lymphoid tissue equivalent (LTE). We used the same approach *in vivo* by putting antigen in a different location, far away from the ICs.

### Example 6. Dual immunization in vitro

A balance between activating/inhibitory FcyRs may regulate signaling in DCs. For example, selective blockade of inhibitory FcyRllB enables human dendritic cell maturation (Dhodapkar et al. (2005) Proc. Natl. Acad. Sci. USA 102, 2910-2915).

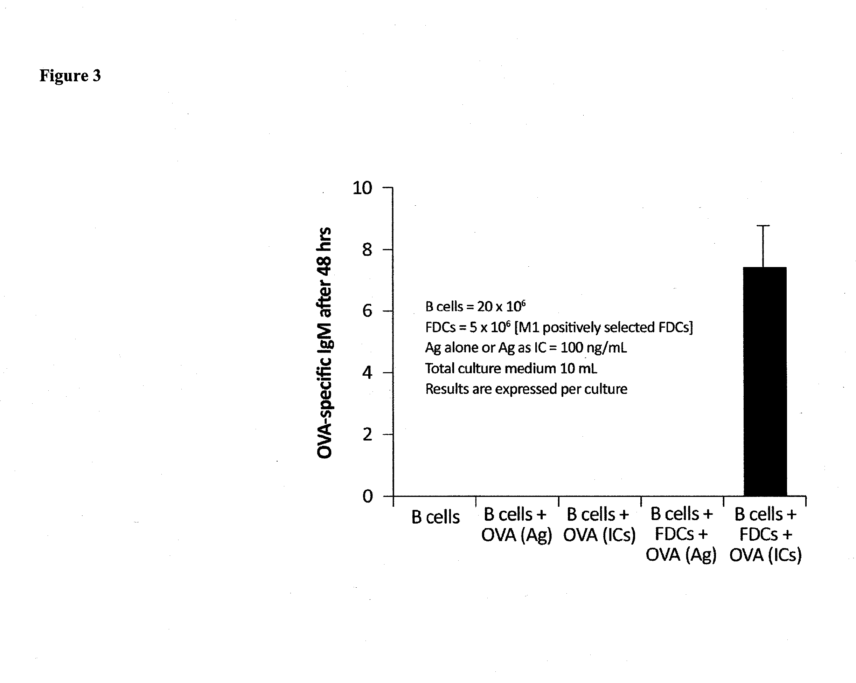

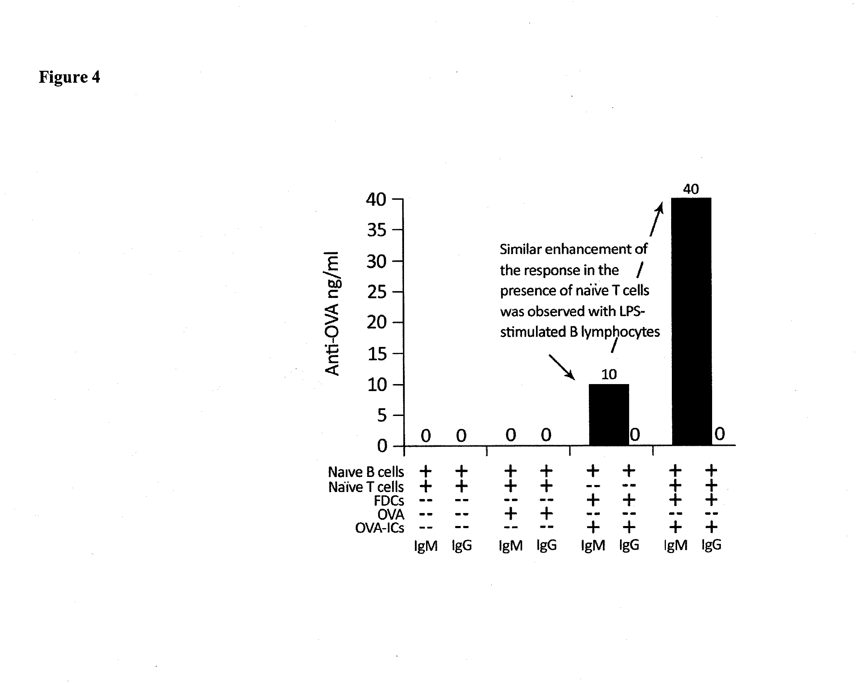

Accordingly, to prime T cells we used free antigen rather than ICs to avoid FcyRllB on DCs. Note the superiority of ICs over free antigen when presented to B cells by FDCs. In the experiment shown in Figure 3, there are no T cells, but there was a specific IgM response with just B cells and FDCs. In Figure 4, the presence of T cells, which likely made some cytokines, did improve the IgM response, but did not result in any IgG, as expected (Fig. 3,4).

### Example 7. Dual immunization in vivo

We tested a dual immunization strategy *in vivo.* ICs were targeted to FDCs to initiate an early IgM response and to expand the specific B cells, while free Ag was injected into a different site to target DCs for T cell priming. In combination, rapid specific IgM responses and enhanced IgG responses were induced and this appeared to be a consistent result (Fig. 5). Furthermore, ICs promoted somatic hypermutation several days earlier in the immune response and this should lead to rapid production of high affinity Abs (Fig. 6, 7).

### Example 8. Purified FDCs can re-attach to an ETC matrix

Purified FDCs can re-attach to an ETC matrix and attract B and T cells to form lymph node-like follicles *in vitro.* We showed that FDCs adhere to collagen and to collagen-associated molecules *in vitro.* Moreover, on collagen type 1, we found that the FDCs would extend dendrites and form FDC-reticula. See El Shikh et al. (2007) Ce// Tissue Res. 329, 81-89. We observed lymph node-like follicles in the GC LTE.

### Example 9. Human B cell responses

To determine whether specific primary *in vitro* human B cell responses could be generated in 3-D *in vitro* GCs, we used an ETC matrix using naïve human B cells and human memory T cells in combination with antigen-bearing FDCs. We observed anti-tetanus responses using memory T cells to induce anti-tetanus responses including responses with lgm-bearing B cells that we believe are naïve. Our success with this model allowed us to examine whether monocyte-derived dendritic cells could be used to prime naïve human T cells and whether these primed T cells could be used to create a complete primary immune response *in vitro.*

The germinal center LTE has been used to generate specific IgM followed by switching to IgG in response to OVA (Fig. 8a). Figure 8b shows the IgG data and is consistent with class switching. After the first week, the IgM response was maximal, with a small IgG response, but by the end of the second week the response had class switched, giving minimal IgM production, and the IgG response was maximal. Additionally, we generated similar data with influenza antigens and anthrax rPA (recombinant protective antigen) (data not shown). However, with HIV gp120 we got a strong IgM response, but failed to get class switching (Fig. 9).

This lack of an IgG response may be attributable to gp120 binding to CD4 and interfering with T cell priming. We attribute the good gp120-specific IgM to the ability of FDCs to arrange lcs on their surfaces with periodicity. This periodicity is consistent with the periodicity of T-independent antigens that give the good IgM responses in the absence of primed T cells.

Simultaneous binding of multiple BCRs gives a signal adequate to give specific IgM responses in the absence of specific T cells. The ability of FDCs plus lcs to induce T-independent responses is illustrated in nude mice, below.

### Example 10. Assessment of potential immunogens

The response in the GC LTE is instructive regarding the use of free gp120 as an immunogen in humans. A common way to assess potential immunogens is to start by injecting them into animals. Those immunogens that cause responses in animals are candidates for further study.

Regarding lg class switching, when gp120 was injected into mice (Fig. 10), the murine response to gp120 was good, with IgM responses that class-switched to IgG by day 14, as expected. There was no indication that gp120 would not be a good vaccine candidate from these murine data.

In short, the GC LTE predicted problems with free, soluble gp120 that can bind human CD4 when priming human T cells, and T cell help is necessary for IgG class switching. In contrast, soluble, free gp120 looks like a good vaccine candidate in an animal model, where IgG class switching occurred normally. It should be appreciated that gp120 will *not* bind *murine* CD4 and would not interfere with T cell priming. Nevertheless, gp120 on the virus *in vivo* does induce a good gp120 response. It may be that use of gp120 in a particle, mimicking the virus, may not block T cell priming as well as the free molecule.

Thus, designing the vaccine differently may give a different result. However, it seems unlikely that free gp120 would be a good immunogen in humans, and only the *in vitro* human artificial immune system of the present invention provided that information.

### Example 11. Assessment of vaccines, in vitro and in vivo

We compared the magnitude and quality of primary and secondary immune responses *in vivo* with responses obtained in the *in vitro* AIS using both established and experimental vaccines. We demonstrated that dual forms of immunogen can lead to a rapid IgM response, as well as a T cell response, leading to class switching and high-affinity IgG production (data not shown).

ICs do not always activate DCs and prime T cells. The dual immunization approach of the present invention targets ICs to FDCs to initiate an early IgM response and expand specific B cells, in combination with antigen targeted to DCs to prime the T cells, resulting in rapid, specific IgM as well as more rapid and enhanced IgG responses.

Priming of naïve T cells requires DCs that are regulated by a balance between activating/inhibitory FcyRs that control signaling in DCs. For example, selective blockade of inhibitory FcyRllB enables human dendritic cell maturation, as was first shown by Dhodapkar et al. (2005) Proc. Natl. Acad. Sci. USA 102, 2910-2915.

In short, ICs may or may not prime T cells and that appears to have frustrated attempts to use ICs in vaccines. The final assessment from a review discussing that fact that ICs often give rapid and enhanced immune responses was that, "[b]ased on reports published to date, it is difficult to predict whether a given antibody will have an enhancing or suppressive effect on the magnitude or efficacy of the subsequent immune response to the antigen." (Brady (2005) Infection & Immunity 73, 671-678).

Given the known problems with ICs in priming naive T cells, in an embodiment of the present invention, we used free antigen rather than ICs to avoid FcyRllB on DCs for the *in vitro* primary. We used the same approach *in vivo.* We injected free antigen into a separate site, to target antigen to a separate lymph node from the ICs, allowing for T cell priming in the absence of ICs. In contrast, with T cells, ICs were much better with FDCs than free antigen *in vitro.* When presented to B cells by FDCs, ICs appear to be much better *in vivo.*

### Example 12. Use of adjuvant to promote a strong antibody response

We have previously shown that FDCs bear TLR4 and other TLRs on their surfaces. Moreover, LPS activates FDCs and enhances their ability to stimulate antibody responses *in vitro* and promote somatic hypermutation.

We next looked to see whether adjuvant could improve the ability of ICs to promote Ab responses (Fig. 11). ICs in adjuvant and ICs alone appeared to have comparable ability to induce OVA-specific IgG. In this example, the ICs were able to induce IgG without there being memory T cells or antigen to prime T cells. Nevertheless, adding adjuvant to the ICs resulted in a dramatic enhancement of the IgG responses, consistent with our data indicating that FDCs have TLR receptors and are activated by engagement of these receptors. Thus, preferably both the antigen and the ICs are in adjuvant when immunizing with the dual immunization approach of the present invention.

We have shown that T-dependent antigens can be converted into T-*independent* antigens by loading them on FDCs in the form of ICs. These results and the adjuvant results above prompted us to examine whether T-dependent antigens could induce IgM in nude mice and whether the IgM response would be enhanced by use of adjuvant. The results of this experiment are illustrated in Figure 12.

OVA in adjuvant failed to induce a detectable IgM response in nude mice, as was expected. In contrast, OVA ICs induced a significant IgM response and that response was enhanced by the use of ICs with the adjuvant.

Thus, in an embodiment of the present invention, ICs are used to provide protection in people with T cell insufficiencies where antigen fails to give a response (as shown here with the nude mice) or a very poor response. Such human immunoinsufficiencies include AIDS patients, the aged, uremics, diabetics, and alcoholics. In an embodiment of the present invention, a method for generating rapid (∼24-48 h) protection is provided by injecting the antigen as an lC.

### Example 13. lC-bearing FDCs

Epitope clusters on FDC dendrites may simultaneously cross-link multiple BCRs; thus, FDCs may convert TD antigens into TI antigens capable of inducing B cell activation and rapid IgM production in the absence of T cells or T cell factors. To test this, lC-bearing FDCs were used to stimulate B cells *in vivo* and *in vitro* under conditions lacking T cell help. Nude mice (nu/nu) were challenged with OVA-ICs and the OVA-specific Abs were measured after ∼48 h (Fig. 13). GC development was also studied in these mice using light and confocal microscopy. Moreover, purified FDCs loaded with OVA-ICs or anti-delta (anti-mouse IgD) ICs were cultured with purified murine and human B cells *in vitro* and the OVA-specific and total IgM responses were measured respectively. Confocal microscopy and flow cytometry were used to visualize and quantify tyrosine phosphorylation indicative of signaling in B cell by lC-bearing FDCs *in vitro.*

Our data indicated that OVA-lC challenged nude mice produced OVA-specific IgM within ∼48 h and the response was maintained for ∼7 weeks (Fig. 13). The draining lymph nodes of these mice exhibited well developed PNA⁺ and GL7⁺ GCs associated with antigen retaining reticula (ARR) and Blimp-1⁺ plasmablasts. Moreover, OVA-IC loaded FDCs induced purified human and murine B cells to produce OVA-specific IgM *in vitro* in ∼48 h. FDCs loaded with anti-delta induced high levels of total IgM within ∼48 h when cultured with purified B cells. Anti-delta IC-stimulated B cells showed characteristic capping and patching of intracellular phosphotyrosine and the intensity of phosphotyrosine labeling was increased in all stimulated B cells as indicated by increased mean fluorescence intensity and total population shift in flow cytometry. FDCs trapped and retained ICs on their surfaces, as shown by confocal microscopy and were able to induce rapid IgM production by purified B cells *in vitro* within ∼48 h. ln short, we have shown the ability of FDCs to convert TD antigens into TI antigens, capable of inducing B cell activation and lg production in the absence of T cells or T cell factors.

Thus, in an embodiment of the present invention, immune responses are induced to TD antigens in patients with congenital and acquired T cell insufficiencies, including infants, the aged, AIDS patients, diabetic, and uremic patients.

### Example 14. Immune response to ovalbumin (OVA)

Homozygous athymic NCr*-nu*/*nu* and heterozygous NCr-nu/+ mice were purchased from The National Cancer Institute at Frederick (NCI-Frederick). Mice were housed in standard plastic shoebox cages with filter tops and maintained under specific pathogen-free conditions, in accordance with guidelines of the Virginia Commonwealth University Institutional Animal Care and Use Committee.

### Challenge of nu/nu mice with OVA immune complexes

Mice were injected with 20 µg ovalbumin (OVA), 5 µg in each limb, in the form of (1) alum precipitated OVA (Sigma-Aldrich, St. Louis, MO, A5503) with *Bordetella pertussis,* or (2) OVA immune complexes (ICs) made of NlP(4-Hydroxy-3-iodo-5-nitrophenylacetyl)-OVA (Biosearch Technologies, Novato, CA, N-5041-10) + goat polyclonal anti-tri-nitro-phenol Abs (Anti-TNP, Biomeda corp., Foster City, CA, J05) or (3) OVA ICs made of alum precipitated NIP-OVA with *Bordetella pertussis* + anti-TNP. Anti-TNP Abs effectively bind the OVA-conjugated NIP forming ICs. Azide-free Functional-Grade Purified anti-mouse CD90 (50 µg, Thy-1, eBioscience, 16-0901) were given IP per mouse to inhibit residual T cell activity, especially γ-δ T cells, that may be present in these animals. Animals were bled after 48 h, 1 week, and 2 weeks. Homozygous *nu*/*nu* mice were also bled after 7 weeks and mid-saggittal sections in the popliteal lymph nodes were labeled for GC B cells with peroxidase-conjugated peanut agglutinin (PNA-HRP, Sigma-Aldrich, St. Louis, MO, L7759). Ova-specific IgM was assessed in the collected sera and levels were recorded after subtracting the pre-immunization background levels.

### Enzyme immunohistochemistry and light microscopy

To test for GC development in OVA-ICs challenged *nu*/*nu* mice, popliteal lymph nodes (LNs) were collected and frozen in CryoForm embedding medium (IEC). Frozen sections of 10 µm thickness were cut on a Leica cryostat (Jung Frigocut 2800E) and air dried. Following absolute acetone fixation, the sections were dehydrated and the endogenous peroxidase activity was quenched with the Universal Block (Kirkegaard & Perry Laboratories, 71-00-61). Mid-saggital sections were washed, saturated with 10% BSA, before incubation with HRP-conjugated PNA. The sections were washed then developed using diaminobenzidine substrate kit (BD Pharmingen, San Jose, CA, 550880). The sections were washed, mounted, and coverslipped; images were captured with an Optronics digital camera on an Olympus light microscope.

### Confocal microscopy

Although they lack reactivity to OVA (Schuurman et al. (1992) J. Exp. Anim. Sci. 35, 33-48), the fact that environmentally-induced GCs have been reported in old-age un-immunized athymic rats (Schuurman et al. (1992) J. Exp. Anim. Sci. 35, 33-48) prompted us to confirm that GCs developing in athymic *nu*/*nu* mice challenged with OVA-ICs co-localize with OVA-ICs retaining reticula and express phenotypic markers characteristics of GCs in normal mice. To test this, two groups of *nu*/*nu* mice were challenged with: a) OVA-specific rabbit serum (Meridian Life Science Inc, Cincinnati, OH, W59413R) plus alum-precipitated OVA and *B. pertussis* or b) normal (non-specific) rabbit serum (Gibco, Grand Islands, NY plus alum-precipitated OVA and *B. pertussis.* Mid-saggittal sections (10 µm-thick) in the axillary lymph nodes were triple labeled with GL7-FITC (BD Pharmingen, San Jose, CA, 553666) (for GC B cells), B220-Cy5.5 (Pharmingen, San Jose, CA, 552771) (general B cell marker), and Rhodamine Red-X-AffiniPure Goat Anti-Rabbit IgG (Jackson ImmunoResearch Laboratories, West Grove, PA, 111-295-144) multiple adsorbed to minimally cross react with mouse, rat and human serum proteins (to label the OVA ICs retained in the FDC reticulum). To label for GC-associated plasmablasts, the Rhodamine Red-X-AffiniPure Goat Anti-Rabbit IgG was replaced in some sections with Blimp-1-PE (Santa Cruz Biotechnology Inc, Santa Cruz, CA. sc-13203 PE). Sections were mounted with anti-fade mounting medium, Vectashield (Vectashield, Vector Laboratories, Burlingame, Calif.), cover-slipped, and examined with a Leica TCS-SP2 AOBS confocal laser scanning microscope fitted with an oil plan-Apochromat 40X objective. Three lasers were used, Argon (488 nm) for FITC, HeNe (543 nm) for Rhodamine-Red X or PE, and HeNe (633 nm) for Cy5.5 (shown as pseudo-color magenta). Parameters were adjusted to scan at 512x512 pixel density and 8-bit pixel depth. Emissions were recorded in three separate channels and digital images were captured and processed with Leica Confocal and LCS Lite software.

### In vitro stimulation of purified B cells with purified IC-bearing FDCs

Naïve untouched human B cells were purified by negative selection on LS MACS separation columns using The Naive B Cell Isolation Kit II (Miltenyi Biotec, Auburn, CA, 130-091-150). Murine B cells were purified by positive selection on LS MACS separation columns using CD45R (B220) MicroBeads (Miltenyi Biotec, Auburn, CA, 130-049-501).

FDCs were isolated by positive selection from LNs (axillary, lateral axillary, inguinal, popliteal, and mesenteric) of irradiated adult mice, as previously described (Sukumar et al. (2006) J. Immunol. Methods 313, 81-95). One day before isolation, mice were irradiated with 1000 rad to eliminate most lymphocytes, and then sacrificed, and LNs were collected, opened, and treated with 1.5 mL of collagenase D (22 mg/ml, C-1088882; Roche), 0.5 mL of DNase I (5000 U/mL, D-4527; Sigma-Aldrich), and 2 mL of DMEM with 20 mM HEPES. After 45 min at 37°C in a CO₂ incubator, released cells were washed in 5 mL of DMEM with 10% FCS. Cells were then sequentially incubated with FDC-specific Ab (FDC-M1) (BD Pharmingen, San Jose, CA, 551320) for 45 min, 1 µg of biotinylated anti-rat L chain (BD Pharmingen, San Jose, CA, 553871), for 45 min, and 20 µL of anti-biotin microbeads (Miltenyi Biotec, Auburn, CA, 130-090-485) for 15-20 min on ice. The cells were layered on a MACS LS column and washed with 10 mL of ice-cold MACS buffer. The column was removed from the VarioMACS, and the bound FDCs were released with 5 mL of MACS buffer.

Purified FDCs were loaded with 100 ng/mL OVA ICs made of OVA/rabbit anti-OVA at a ratio of 1:6. lC-loaded FDCs were used to stimulate 20x10⁶ purified B cells at a ratio of 1 FDC:2B cells. Cells were cultured in 10 mL culture medium and OVA-specific Abs were assessed after 48 h.

The rat anti-mouse IgD mAb clone 11-26 (SouthernBiotech, Birmingham, Alabama, 1120-14) was complexed with Fc-specific rabbit anti-rat IgG (Jackson ImmunoResearch Laboratories, West Grove, PA, 312-005-046) at a ratio of 1:4 and ICs were used to load purified FDCs or FDC-like cells. This monoclonal antibody *per* se does not induce proliferation of mature B cells *in vitro,* nor does *in vivo* injection of the monoclonal antibody have any effect on activation of B lymphocytes. FDCs and FDC-like cells were loaded with anti-delta ICs at doses of 0.1, 1.0, and 10 µg/mL and used to stimulate 10⁴, 10⁵, and 10⁶ purified murine B cells in 1 mL cDMEM. Culture supernatants were assessed after 48 h for total mouse IgM production using ELISA.

### ELISA

Total and OVA-specific IgM were assessed in sera and culture supernatants ∼48 hours after stimulation of B cells with OVA or anti-lgD IC-bearing FDCs in vivo and in vitro. Samples were loaded on 96-well plates coated with 100 µg/mL OVA (for OVA-specific Abs) or goat anti-mouse IgM (for total IgM). Samples were left overnight, washed and captured mouse IgM was detected with biotinylated goat anti-mouse IgM followed by streptavidin-alkaline phosphatase. Alkaline phosphatase was developed with pNPP alkaline phosphatase substrate system (KPL, Gaithersburg, Maryland, 50-80-00) and read on ELISA reader at 405 nm.

### Visualization and quantification of intracellular phosphotyrosine in stimulated B cells using confocal microscopy and flow cytometry

Purified B cells were stimulated with anti-lgD IC-bearing FDCs for 45 min. Cells were washed, fixed, and permeabilized using the Fix & Perm cell permeabilization kit (Caltag Laboratories). Intracellular phosphotyrosine was detected with FITC-conjugated Anti-Phosphotyrosine, clone 4G10, (Upstate Biotechnology, Lake Placid, NY, 16045). Cells were washed and analyzed with flow cytometry or deposited onto polylysine-coated glass slides for visualization with confocal microscopy using argon beam emitting 488 nm laser.

### Nude mice challenged with OVA ICs, but not OVA, developed GCs and OVA-specific IgM

lf periodically arranged FDC-ICs can induce specific IgM in the absence of T cells, then nude mice should rapidly produce specific IgM when challenged with a TD antigen in the form of ICs but not with TD antigen alone. This hypothesis was tested in *nu*/*nu* mice given 500 µg of α-Thy-1, to block any residual T cell activity, and challenged with OVA in adjuvant or OVA in ICs with or without adjuvant. As expected, anti-OVA was not detectible in animals immunized with OVA over a 7-week period, even with adjuvant. (Fig. 13). In marked contrast, OVA-specific IgM was present in the sera of all ICs-injected animals with or without adjuvant in just ∼48 h. The highest OVA-lgM levels were induced using adjuvant-supplemented OVA-ICs and these IgM levels were maintained over a 7 week assessment period.

This is not unexpected, as LPS will activate FDCs and promote their accessory activities (El Shikh et al. (2007) J. Immunol. 179, 4444-4450). Well-developed PNA⁺ GCs were observed in the draining lymph nodes of the lC-challenged animals, further supporting FDC-mediated B cell activation (Fig. 13). Phenotypically normal heterozygous nu/+ mice also responded to ICs by producing OVA-specific IgM within ∼48 h (Fig. 13), although, these IgM levels declined as the isotype switched from OVA specific IgM to IgG, in the presence of T cell help (Fig. 13).

### lC-induced GCs in nude mice are associated with well-developed ARR and plasmablasts

As expected for a T-dependent protein antigen, GCs were not detected in athymic nude mice, nu/nu mice challenged with OVA (data not shown). The B cell follicles labeled with B220, but not with the GC B cell marker GL7. In marked contrast, the follicles in *nu*/*nu* mice challenged with OVA-ICs developed large GCs. In an overlay, GL7 bright GC B cells surrounded by a zone of un-activated B220 bright B cells were seen. There was an area of dim B220 labeling. Activated B cells tend to downregulate B220 and this dim B220 area correlated with the expression of the activation marker GL7. ln some images, a well-developed crescent-shaped ARR labeled with anti-rabbit IgG (identifies the rabbit IgG in trapped ICs made of OVA + rabbit-anti-OVA IgG) was seen. A funnel-shaped antigen transport site extending from the sub-capsular sinus into the lymph node cortex was apparent.

### Purified OVA IC-bearing FDCs induced OVA-specific IgM production by purified B cells within ∼48 h in the absence of T cells and T cell factors

lf periodically arranged FDC-ICs can induce specific IgM in the absence of T cells, then purified FDCs, bearing a TD antigen in the form of ICs, but not with TD antigen alone, should rapidly stimulate specific IgM by naïve B cells *in vitro.* FDC-B cell interactions are not MHC or species restricted and murine FDCs can stimulate human B cells effectively (Fakher et al. (2001) Eur. J. Immunol. 31, 176-185). Purified murine (Fig. 14A) or human (Fig. 14B) B cells stimulated with FDCs bearing OVA lC in cultures lacking T cells and T cell factors produced OVA-specific IgM in ∼48 hours. Both the kinetics of the response and the IgM production were consistent with a T-independent response. Control conditions, that failed to produce a detectable response, included FDCs with B cells stimulated with free OVA that would have had unfettered access to BCR.

### Purified B cells were signaled by FDCs bearing anti-IgD ICs as indicated by increased levels and distribution of intracellular phosphotyrosine

Extensive cross linking of BCRs can lead to B cell signaling, as indicated by increases in and redistribution of intracellular phosphotyrosine in caps and patches. We sought for evidence that ICs arranged on FDCs can signal B cells. We reasoned that anti-lgD loaded on FDCs in the form of ICs should engage multiple BCRs and induce B cell phosphotyrosine in caps and patches near the membrane surface. The anti-lgD mAb (rat anti-mouse IgD clone 11-26) was selected for this study because it does not induce B cell activation. This mAb was complexed with Fc-specific rabbit anti-rat IgG (to leave the Fabs free to engage BCRs) and loaded on the surface of FDCs. Phosphotyrosine labeling in unstimulated B cells was low and evenly distributed. In contrast, B cells stimulated with FDCs bearing ICs labeled more intensely and the phosphotyrosine was capped (fluorescence localized at one pole of the cell surface), or patched on the membrane indicating a marked redistribution (data not shown). Most B cells exhibited the patched or capped intracellular phosphotyrosine pattern consistent with being signaled. Moreover, flow cytometric analysis confirmed that the B cells exhibited higher levels of intracellular phosphotyrosine (increased MFI) and the entire B cell population had clearly shifted to the right suggesting that virtually the entire B cell population had been signaled by anti-delta bearing FDCs. Higher magnification imaging revealed that the phosphotyrosine labeling was intense at areas of contact between the B cell membrane and the AMCA-labeled lC-bearing FDCs.

### Purified B cells stimulated with FDCs bearing anti-IgD ICs on their surfaces produced IgM within 48 h

Given that B cells are signaled by anti-delta ICs on FDCs, we reasoned that the simultaneous engagement of multiple B cell receptors should signal, at least some of these B cells adequately, to rapidly produce IgM. Moreover, this should be possible in the absence of T cells, as was seen in Figure 14 where OVA ICs induced OVA-specific IgM responses *in vitro* in the absence of T cells or T cell factors. Accordingly, we sought to test the hypothesis that anti-BCR bearing FDCs induce substantial polyclonal IgM responses in the absence of T cells or T cell factors. As shown in Figure 15, ∼1×10⁴-∼1×10⁶ B cells stimulated with 0.1-10 µg/mL anti-lgD ICs loaded on FDCs produced IgM within ∼48 h in a B cell dose-dependent fashion, although ∼100 ng of lC stimulated as well as 10 µg. In the absence of FDCs, anti-lgD ICs did not induce production of IgM, even at doses of 10 µg/mL.

T-I type 2 Ags show periodically arranged epitopes attached to a flexible backbone. Their structure allows extensive cross-linking of BCRs and activation of B cells. Although T-D Ags possess multiple epitopes on their surfaces, each particular epitope is not repeatedly presented and accordingly BCRs specific for that epitope are not cross-linked and B cells are not activated. We believe that, if T-D Ags can be spatially approximated so that similar epitopes are close enough to cross-link multiple BCRs specific for the epitope, B cells can be activated without the need for T cell help.

FDCs express high levels of FcyRIIB and CRs, which trap ICs containing TD Ags (multi-color clusters) that are periodically arranged on FDCs with ∼100-500 Å spacing that is ideal for extensive BCR cross-linking and B cell activation. Transmission electron micrographs showed HRP (TD Ag) loaded on the surface of FDCs as ICs with distances between IC clusters ranging between ∼200-500 Å this arrangement is fit to cross-link BCRs and activate B cells.

FDCs can stimulate B cells not only by cross-linking their BCRs, but secondary accessory signals can also be delivered. As detailed previously FDCs provide a complement-derived CD21 L for B cell CD21; its interaction with the CD21-CD19-CD81 complex delivers a positive co-signal for B-cell activation and differentiation (Tew et al. (2001) Trends Immunol. 22, 361-367; Fakher et al. (2001) Eur. J. Immunol. 31, 176-185; Qin et al. (1998) J. Immunol. 161, 4549-4554; Qin et al. (1997) Adv. Exp. Med. Biol. 417, 493-497; Qin et al. (2000) J. Immunol. 164, 6268-6275; Aydar et al. (2004) Eur. J. Immunol. 34, 98-107; Aydar et al. (2003) J. Immunol. 171, 5975-5987). Coligation of BCR and CD21 facilitates association of the two receptors, and the cytoplasmic tail of CD19 is phosphorylated by a tyrosine kinase associated with the BCR complex. Additionally, the high density of FcyRllB on FDCs binds Ig Fc in the Ag/Ab complex saving the B cells from the inhibitory signal delivered by the immunoreceptor tyrosine-based inhibition motif (lTlM) if the ICs were left to cross-link the BCR and the FcyRllB on B cells. FDC-derived BAFF (Hase et al. (2004) Blood 103, 2257-2265; Ng et al. (2005) Mol. Immunol. 42, 763-772) that ligates BAFF receptors on B cells, and FDC-derived C4b-binding protein (C4BP) (Gaspal et al. (2006) Eur. J. Immunol. 36, 1665-1673) that ligates CD40 on B cells are other molecules that can deliver accessory activation signals to the B cells.

Without wanting to be bound by any mechanism, from these experiments, we propose an FDC-dependent T cell-independent multi-signal model for B cell activation and lg production. FDCs deliver a first BCR-mediated signal via extensive cross-linking of multiple BCR clusters helped by the flexibility of FDC dendrites that can geometrically fit the contour of B cells, in addition to FDC-derived accessory signals, known for their ability to co-stimulate B cells (see Fig. 1).

All documents, publication, manuals, article, patents, summaries, references and other materials cited herein are incorporated herein by reference in their entirety. Other embodiments of the invention will be apparent to those skilled in the art from consideration of the specification and practice of the invention disclosed herein. It is intended that the specification and examples be considered as exemplary only, with the true scope and spirit of the invention being indicated by the following claims.

## Claims

1. A two-component vaccine system comprising a first component and a second component, wherein the first component comprises an antigen and wherein the second component comprises an immune complex of the antigen or portion thereof of the first component, preferably wherein the first component further comprises a pharmaceutically acceptable carrier or diluent and the second component further comprises a pharmaceutically acceptable carrier or diluent.

2. A two-component vaccine system for use in inducing an immune response in a subject comprising:
(a) a first component of the two-component vaccine system for administration to a subject, wherein the first component comprises an antigen and pharmaceutically acceptable carrier or diluent, and
(b) a second component of the two-component vaccine system for administration to the subject, wherein the second component comprises an immune complex of the antigen or portion thereof of the first component and a pharmaceutically acceptable carrier or diluent, preferably wherein said immune response is a protective immune response.

3. The system of claim 2, wherein the second component of the two-component vaccine system is for administration to a different location of the subject than the first component of the two-component vaccine system.

4. The system of claim 2 or 3, wherein the first and second components of the two-component vaccine system are for administration concurrently or sequentially to the subject.

5. The system of any one of claims 2 to 4, wherein the immune response is a rapid production of high-affinity antibodies, preferably wherein the high-affinity antibodies are high-affinity IgM antibodies or high-affinity IgG antibodies, further preferably wherein the high-affinity antibodies are produced within about 24 hours after administration of the two-component vaccine system.

6. Use of a two-component vaccine system in the manufacture of a medicament for inducing an immune response in a subject comprising:
(a) a first component of the two-component vaccine system for administration to a subject, wherein the first component comprises an antigen and pharmaceutically acceptable carrier or diluent, and
(b) a second component of the two-component vaccine system for administration to the subject, wherein the second component comprises an immune complex of the antigen or portion thereof of the first component and a pharmaceutically acceptable carrier or diluent, preferably wherein said immune response is a protective immune response, wherein preferably said system is as defined in any one of claims 2 to 4.

7. The system or use of any one of claims 1-6, wherein the antigen of the first component is a polypeptide, peptide, protein, polysaccharide, an attenuated bacteria or an attenuated virus, preferably wherein said protein is a bacterial surface protein, a bacterial integral membrane protein, or a viral coat protein.

8. The system or use or use of any one of claims 1-7, wherein the antigen is derived from a human disease-causing bacteria or virus.

9. The system or use of any one of claims 1-8, wherein the immune complex of the second component comprises an antibody and the antigen or portion thereof to which the antibody is bound.

10. The system or use of claim 9, wherein the antibody is a polyclonal antibody, monoclonal antibody or chimeric antibody, preferably a human antibody.

11. The system or use of claim 9 or 10, wherein the antibody is selected from the group consisting of IgG1, lgG2, lgG3, lgG4, IgM, IgA1, IgA2, IgD and IgE antibodies.

12. The system or use of any one of claims 9-11, wherein the antibody is an antibody fragment which retains the ability to bind the antigen selected from the group consisting of a single chain antibody, a F(ab')2 fragment, and a Fab fragment.

13. The system or use of any one of claims 1-12, wherein the immune complex of the second component comprises a portion of the antigen of the first component.

14. The system or use of any one of claims 1-13, wherein the antigen or portion thereof of the second component is a peptide of 10-15 amino acids.

15. The system or use of any one of claims 9-14, wherein the antibody and the antigen or portion thereof of the second component are chemically coupled.
